# EUROPEAN PATENT SPECIFICATION

(11) **EP 2 203 184 B1**
(45) Date of publication and mention of the grant of the patent: **14.03.2012**
(21) Application number: 08804566.1
(22) Date of filing: 22.09.2008
(51) Int. Cl.: A61K 39/245

(54) **VACCINE COMPOSITION FOR THE PREVENTION OF CMV INFECTIONS**
IMPFSTOFFZUSAMMENSETZUNG ZUR PRÄVENTION VON CMV-INFEKTIONEN
COMPOSITION VACCINALE POUR LA PRÉVENTION D'INFECTIONS À CMV

(30) Priority: 21.09.2007 US 974231 P
(43) Date of publication of application: 07.07.2010
(73) Proprietor: Sanofi Pasteur, 69367 Lyon Cedex 07 (FR); The U. A. B. Research Foundation, Alabama, AL35294 (US)
(72) Inventor: PASS, Robert F., Birmingham, AL 35213 (US); CLOUD, Gretchen, Birmingham, AL 35242 (US)
(74) Representative: Hurpin, Christian Marcel
(86) International application number: PCT/EP2008/062640
(87) International publication number: WO 2009/037359

(56) References cited:
- MARSHALL G S: "Cytomegalovirus vaccines - Two decades of progress" HERPES 1997 GB, vol. 4, no. 1, 1997, pages 20-24, XP008099355 ISSN: 0969-7667
- DATABASE BIOSIS [Online] BIOSCIENCES INFORMATION SERVICE, PHILADELPHIA, PA, US; 1995, HARRISON CHRISTOPHER J ET AL: "Reduced congenital cytomegalovirus (CMV) infection after maternal immunization with a guinea pig CMV glycoprotein before gestational primary CMV infection in the guinea pig model" XP002506912 Database accession no. PREV199698564640 & JOURNAL OF INFECTIOUS DISEASES, vol. 172, no. 5, 1995, pages 1212-1220, ISSN: 0022-1899
- MARSHALL G S ET AL: "Safety and immunogenicity of CMV gB/MF59 vaccine in healthy seronegative adults" ABSTRACTS OF THE INTERSCIENCE CONFERENCE ON ANTIMICROBIAL AGENTS AND CHEMOTHERAPY, vol. 37, 1997, page 228, XP008099210 & 37TH INTERSCIENCE CONFERENCE ON ANTIMICROBIAL AGENTS AND CHEMOTHERAPY; TORONTO, ONTARIO, CANADA; SEPTEMBER 28-OCTOBER 1, 1997
- PODDA A ET AL: "MF59-adjuvanted vaccines: Increased immunogenicity with an optimal safety profile" EXPERT REVIEW OF VACCINES 200304 GB, vol. 2, no. 2, April 2003 (2003-04), pages 197-204, XP008099203 ISSN: 1476-0584
- BERNSTEIN DAVID I ET AL: "Effect of previous or simultaneous immunization with canarypox expressing cytomegalovirus (CMV) glycoprotein B (gB) on response to subunit gB vaccine plus MF59 in healthy CMV-seronegative adults." THE JOURNAL OF INFECTIOUS DISEASES 1 MAR 2002, vol. 185, no. 5, 1 March 2002 (2002-03-01), pages 686-690, XP002506911 ISSN: 0022-1899
- MITCHELL DOUGLAS K ET AL: "Immunogenicity of a recombinant human cytomegalovirus gB vaccine in seronegative toddlers." THE PEDIATRIC INFECTIOUS DISEASE JOURNAL FEB 2002, vol. 21, no. 2, February 2002 (2002-02), pages 133-138, XP008099162 ISSN: 0891-3668
- GONCZOL E ET AL: "DEVELOPMENT OF A CYTOMEGALOVIRUS VACCINE: LESSONS FROM RECENT CLINICAL TRIALS" EXPERT OPINION ON BIOLOGICAL THERAPY, ASHLEY, LONDON, GB, vol. 1, no. 3, 1 May 2001 (2001-05-01), pages 401-412, XP008040188 ISSN: 1471-2598

## Description

### Field of the Invention

The invention relates to vaccines for preventing HCMV infection in HCMV seronegative human subjects and for preventing HCMV congenital infection in newborns.

### Summary of the Related Art

The Human cytomegalovirus (HCMV) is a ubiquitous virus belonging to the Herpes virus family. The virus is composed of a linear double-stranded deoxyribonucleic acid (DNA) contained in a capsid surrounded by a tegument and enveloped in a lipid bilayer carrying glycoprotein spikes on its surface. Like other members of this family, HCMV possesses the characteristics of latency and reactivation. HCMV has the ability to infect and be latent in many cells.

In the immunocompetent host, most HCMV infections are asymptomatic or very mild with a few nonspecific symptoms such as fatigue, malaise, moderate fever, lymphadenopathy, hepatomegaly or a slight increase in liver enzymes. Heterophil-negative mononucleosis is however observed in approximately 10% of previously healthy individuals.

In contrast, clinical manifestations can be very severe in newborns infected in utero and in adults immunosuppressed by AIDS or in the context of solid organ or bone marrow transplantation.

The prevalence of HCMV infection increases with age and is affected by socioeconomic factors. Serological surveys have shown a higher prevalence in developing countries and in lower socioeconomic groups of developed countries. For women of child-bearing age, the proportion of HCMV seropositive women ranges from approximately 50% in upper and middle incomes groups of developed countries to over 80% in low-income populations. Surveys performed in different western European countries within the two last decades on the general population including different age-classes, females and males showed globally that HCMV seroprevalence in toddlers and adolescents ranges between 40 and 50% while in older subjects (40 years and over), HCMV seroprevalence is higher than 80%.

HCMV is shed for a prolonged period in the secretions of infected individuals including urine, saliva, milk, semen, genital secretions; HCMV is thus transmitted either horizontally (through intimate contact from child to child, from child to parents and between sex partners) or transplacentally (from mother to infant) or by exposure to blood products or transplanted organs.

HCMV is the most common cause of congenital infection in the developed world. Congenital infection refers to infection transmitted from mother to fetus prior to birth of the newborn. Each year in the United States, an estimated 8000 infants suffer disabilities, including mental retardation, blindness and sensorineural deafness, as a result of congenital HCMV infection.

Among congenitally infected newborns, 5% to 10% have major manifestations at birth such as microcephaly, chorioretinitis, intracranial calcifications, hepatosplenomegaly, hepatitis, jaundice, direct hyperbilirubinemia, thrombocytopenia, petechiae, and anemia. Among these newborns with symptomatic congenital HCMV disease, the mortality rate is approximately 10% in early infancy and among survivors, 50-90% will have sequelae such as mental retardation, cerebral palsy, sensorineural hearing loss or visual impairment.

Many infants with congenital HCMV infection are asymptomatic at birth. Follow-up studies have shown that approximately 15% of infants who are asymptomatic at birth and identified as HCMV seropositive in the newborn period by virological screening will have sequelae such as hearing loss or central nervous system abnormalities.

As a whole, approximately 17,000 infants born each year in Europe and in the USA will have permanent sequelae.

Congenital HCMV infections are more frequent and more severe when the primary infection occurs in the first trimester of pregnancy than when primary infection occurs in pregnancy. Overall, a primary HCMV infection during pregnancy is associated with a 40% risk of transmission to the fetus.

In studies conducted between 1978 and 1984, Stagno et al. [JAMA: 256; 1904-1908 (1986) and New England Journal of Medicine: 306; 945-949 (1982)] monitored 12,140 women for HCMV acquisition during pregnancy. They observed 33 women infected between the 4th and 22nd week after conception. Of these 33 women, over half (17 women in total) gave birth to infected infants. Of the 17 neonates congenitally infected, 5 (29%) had significant handicaps - three were mentally retarded and two were deaf. In 1992, Fowler et al. [New England Journal of Medicine: 326; 663-667 (1992)] found that sequelae occurred in 25% of 125 infants with congenital infection who were born of mothers with a primary HCMV infection during pregnancy. This was contrasted with an 8% rate for 64 infants with congenital infection born of mothers who were HCMV seropositive prior to pregnancy. More importantly, none of the infected infants born of seropositive mothers developed severe sequelae, defined as bilateral hearing loss or mental retardation (an I.Q. < 70). These sequelae occurred only among infants born of mothers who had a primary infection during pregnancy. Such observations suggest that maternal immunity to HCMV prior to pregnancy will prevent the majority of severe sequelae associated with congenital infection.

Effective means of preventing or treating maternal HCMV infection during pregnancy or congenital HCMV infection are currently not available.

HCMV is also an important viral pathogen in organ and bone marrow transplant recipients and in AIDS patients. The rate of HCMV-associated morbidity in HCMV seronegative solid organ transplant recipients approaches 60%. In solid organ transplant the disease is the most severe when seronegative patients receive a graft from a HCMV positive donor. In contrast, in bone marrow or stem cell transplantation the disease is most severe in HCMV seropositive subjects receiving cells from a seronegative donor showing that the origin of HCMV infection is reactivation of endogenous infection.

HCMV causes pneumonitis, hepatitis, gastrointestinal disease, bone marrow suppression, and retinitis in approximately 15% of allograft recipients. In addition to these direct end-organ diseases, HCMV has been associated with indirect effects such as graft rejection, accelerated atherosclerosis and immunosuppression that can lead to bacterial or fungal infection. Cytomegalovirus end-organ disease in allograft recipients is less severe in those with pre-existing immunity (i.e., HCMV seropositive pre-transplant). Specific anti-HCMV hyperimmune globulins have been shown to decrease the severity of HCMV infection in HCMV seronegative recipients of HCMV infected organs [New England Journal of Medicine: 317; 1049-1054 (1987)]. There is thus the potential for vaccines to mimic the protection afforded by natural immunity or by the infusion of hyperimmune globulins and to reduce the use and cost of antiviral drugs given either prophylactically to all patients or pre-emptively to those in whom virus is detected post transplant.

To address the extreme severity of HCMV associated diseases candidate vaccines are being developed. Among them, a live attenuated vaccine based on the Towne strain induces immunity, including neutralizing antibodies and cell-mediated immunity [Pediatric Infectious Diseases Journal: 17; 200-206(1998)]. However, the immune response lags behind that of a natural infection. To increase the immunogenicity of the Towne live attenuated vaccine, which lacks nineteen open reading frames (ORF) and has lost its capacity to establish latency, some of the ORF of the more virulent Toledo strain of HCMV have been introduced into the Towne vaccine strain to produce a chimeric, live virus vaccine [Journal of Infectious Diseases: 193; 1350-1356 (2006)].

Another candidate vaccine being tested is a sub-unit candidate vaccine comprising the envelope gB glycoprotein of HCMV and a canarypox vector containing the pp65 and IE1 exon 4 that are internal proteins of HCMV [Journal of Infectious Diseases: 183; 1171-1179 (2001) and Journal of Infectious Diseases: 185; 686-690 (2002)]. Yet another candidate HCMV vaccine that has reached clinical trials is based on injection of plasmid DNA expressing genes for gB and pp65 [Journal of Infectious Diseases: 197; 1634-1642, (2008)].

Another subunit HCMV vaccine comprising a modified gB glycoprotein combined with the MF59^{™} emulsion was tested in a phase I clinical trial [The Journal of Infectious Diseases: 180; 970-975 (1999)]. Participants received HCMV gB vaccine with MF59^{™} at 0, 1 and 6 months. The levels of neutralizing antibody and antibody to gB two weeks after the third dose exceeded those in seropositive control subjects but there is no evidence that such composition is being able to prevent HCMV infection.

A vaccine comprising gB-mF59 showing good tolerance and high immunogenicity, with theoretic potential, is known from Pediatr. Infect. Dis J, 2002, 21, 133-8

To prevent HCMV infection, a common approach suggests that a HCMV vaccine should mimic the immune response mediated by the natural infection. Essentially, the immune response mediated by the natural infection involves both a cellular immune response and a humoral response directed against the internal structural and regulatory proteins of HCMV (the major matrix protein, phosphoprotein 65 (pp65), Immediate Early 1 (IE1)), and the external proteins of HCMV (gB, gH, gM and gN glycoproteins). The specific cellular immune response, including in particular the CTL response, is directed predominantly against the internal structural and regulatory proteins, whereas the specific humoral response is above all directed against the external proteins. Therefore, a subunit vaccine should normally contain at least one internal protein or a derivative thereof and one external protein or a derivative thereof to be efficient in the prevention of HCMV infection.

### Brief Summary of the Invention

Unexpectedly, it has now been found that a HCMV vaccine composition, although it does not mimic the immune response mediated by the natural infection, is efficacious in the prevention of HCMV infection in HCMV seronegative human subjects and the prevention of HCMV congenital infection in newborns.

The present invention relates to :
1. A recombinant gB subunit vaccine composition containing the recombinant gB antigen of Human Cytomegalovirus (HCMV) in an oil in water (O/W) emulsion for use in a method to prevent HCMV infection in a HCMV seronegative human subject.
2. A vaccine according to 1 for the use according to 1, wherein the seronegative human subject is a HCMV seronegative child-bearing-age woman.
3. A vaccine according to 1 for the use according to 1, wherein the seronegative human subject is a HCMV seronegative mother.
4. A recombinant gB subunit vaccine composition containing the recombinant gB antigen of Human Cytomegalovirus (HCMV) in an oil in water (O/W) emulsion for use in a method to prevent HCMV congenital infection in a newborn.
5. A vaccine according to 4 for the use according to 4, wherein the newborn is born to a HCMV seronegative child-bearing-age woman.
6. A vaccine according to 4, for the use according to 5, wherein the HCMV seronegative child-bearing-age woman is a HCMV seronegative mother.
7. A vaccine according to 1 or 4 for the use according to any of 1 to 6. wherein the gB antigen is the sole antigen of HCMV in the vaccine composition.
8. A vaccine according to 1 or 4 for the use according to any of 1 to 7, wherein the gB antigen of HCMV further comprises one or several mutations on the cleavage site.
9. A vaccine according to 1 or 4 for the use according to any of 1 to 8, wherein the gB antigen of HCMV is a full length gB polypeptide, a full length gB polypeptide lacking substantially all the trans membrane domain, a full length gB polypeptide lacking substantially all the intracellular domain, or a full length gB polypeptide lacking substantially both the transmembrane domain and the intracellular domain.
10. A vaccine according to 1 or 4 for the use according to any of 1 to 9, wherein the gB antigen of HCMV is gBdTm.
11. A vaccine according to 1 or 4 for the use according to any of 1 to 10, wherein the O/W emulsion is a MF59-like emulsion.
12. A vaccine according to 1 or 4 for the use according to any of 1 to 11, wherein the O/W emulsion further comprises a TH-I adjuvant.
13. A vaccine according to 1 or 4 for the use according to any of 1 to 12, wherein the gB antigen of HCMV is to be administered to the human subject in an amount of 5µg to 100 µg.
14. A vaccine according to 1 or 4 for the use according to any of 1 I to 13, wherein the vaccine composition is a ready to be administered formulation, wherein the gB antigen and optionally the TH-1 adjuvant is already mixed within the O/W emulsion.
15. A vaccine according to 1 or 4 for the use according to any of 1 to 13, wherein the vaccine composition is prepared extemporaneously by mixing the O/W emulsion with the gB antigen and optionally with the TH-1 adjuvant prior to administering to the HCMV seronegative human subject.
16. A vaccine according to 1 or 4 for the use according to any of to 15, wherein the vaccine composition is to be administered at least two times within 1-2 month interval between each administration by the subcutaneous (SC), intradermal (ID) or intramuscular (IM) route.
17. A vaccine according to 1 or 4 for the use according to 16, wherein the vaccine composition is administered a third time within a 4 to 6 month interval between the second administration and the third administration.
18. A vaccine according to 1 or 4 for the use according to 17, wherein the vaccine composition is to be administered a fourth time as a booster dose.

### Brief Description of the drawings

Fig 1: represents the Kaplan-Meyer curves showing the probability of remaining HCMV negative in the placebo group and in the vaccine group, in the ITT population.
Fig 2: represents the evolution of the geometric mean titers (GMT) of neutralizing antibodies against HCMV during the 42 months of follow up in a subgroup of 50 uninfected mothers having received 3 vaccine doses at M0, M1 and M6.

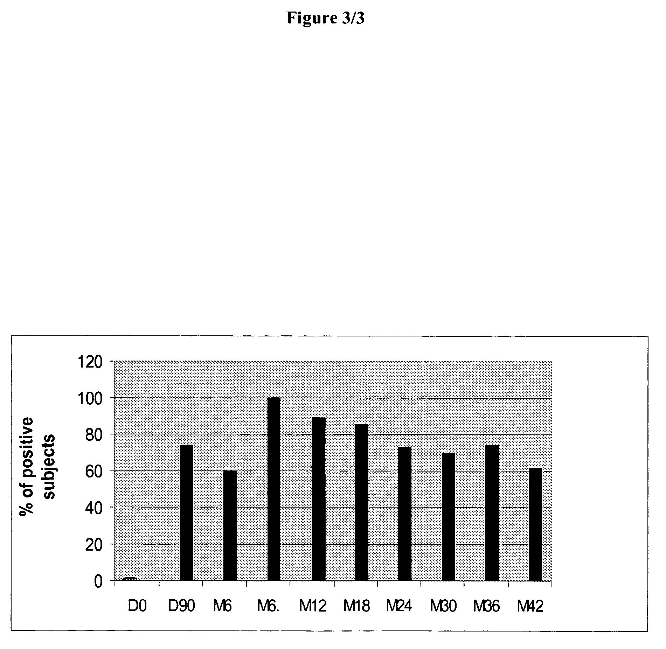

Fig 3: represents the evolution of the percentage of mothers with neutralizing antibodies against HCMV during the 42 months of follow up in a subgroup of 50 uninfected mothers having received 3 vaccine doses at M0, M1, and M6.

### Detailed description of the invention

The present invention provides a vaccine for preventing HCMV infection in HCMV seronegative human subjects, which vaccine composition contains as antigen the gB of HCMV in an O/W emulsion (oil in water emulsion). Unexpectedly, we have found that a gB formulated in an O/W emulsion is efficacious in the prevention of HCMV infection in HCMV seronegative human subjects and, in particular, in young mothers. The vaccine efficacy is more than 70% in the first year that follows the first administration, is still about 60% in the second year that follows the first administration and still remains around 50 % over a 42 months period. Furthermore, it is not necessary to combine the gB antigen with one or several other internal antigens of HCMV (such as pp65, IE1) or even with other external proteins (such as gH, gM and/or gN) to get a significant protection against HCMV infection.

According to one aspect, the vaccine composition for use according to the present invention can contain one or several antigens of HCMV formulated in an O/W emulsion provided that there is at least the gB antigen. By combining the gB antigen with other HCMV antigens (such as gH, gM, gN, pp65, IE1, etc.), one can increase the level and/or the duration of the gB-mediated protection against HCMV.

According to another aspect, the vaccine composition for use according to the present invention contains only the gB antigen as HCMV antigen formulated in an O/W emulsion since it has been shown that gB alone is enough to significantly protect young mothers against HCMV infection.

Even though the vaccine provided by the invention can be administered to the seronegative human male population, it is primarily intended to prevent HCMV infection in the group of HCMV seronegative women of child-bearing age because there is a high risk of HCMV infection in the offspring if a primary HCMV infection occurs during the pregnancy. Usually, the group of HCMV seronegative women of child bearing-age is represented by HCMV seronegative women who are between 14 and 40 years of age that can become pregnant. This group includes both seronegative women that have already been pregnant (mothers) and seronegative nullipara women.

The risk of HCMV transmission to the offspring is higher in HCMV seronegative women who become pregnant before 25 years of age [Journal of Infectious disease 168:552-556, (1993); Journal of Medical Virology 13: 347-353 (1984)]. The risk of HCMV transmission to the fetus is also very high in HCMV seronegative mothers: the high level of seroconversion rate observed in the population of young mothers could be due to their close contact with their infants or toddlers who are exposed to HCMV carriers in day-care centers; an increased rate of HCMV infections has been shown among mothers of children attending day-care centers [New England Journal of Medicine: 321; 1414-1418 (1986)]. In a recent study, a rate of congenital infection of 12.7% was found in the offspring of mothers who seroconverted during the interval between two deliveries (average 3 years) [JAMA: 289; 1008-1011 (2003)].

Therefore, there is also an urgent need to provide the HCMV seronegative mother population with means that prevent HCMV infection between two deliveries. Usually, the interval between two deliveries (especially in the population of mothers who have only one child) is quite short (about 2 to 3 years). The subject of the invention (according to which the vaccine efficacy rate is at least 70% for at least a one-year period and that affords a significant protection against HCMV infection for at least a 42 month period) is therefore especially suitable for the HCMV seronegative mother population and, in particular, for the HCMV seronegative mother population who has only one baby because very often a second pregnancy occurs quickly (in general in a few years after the first pregnancy). The invention is also particularly suitable for the seronegative population of mothers who are less than 25 years old and expose their offspring to the highest risk of congenital infection.

Therefore, the vaccine provided by the invention is in particular especially well suited to prevent HCMV infection in the group of HCMV seronegative population of mothers, more specifically in the group of HCMV seronegative population of mothers who have only one baby and still more specifically in the population of HCMV seronegative mothers who are less than 25 years old (young HCMV seronegative mothers).

The vaccine provided by the invention is also well suited in HCMV seronegative women of child bearing age with the intention to procreate shortly. Indeed, more and more women who have such intention have an appointment, a "preconception visit", with their health care provider shortly before trying to conceive. Such population of women represents therefore a very good target population within the HCMV seronegative women of child bearing age, for which the vaccine provided by the invention is particularly advisable for the prevention of HCMV infection and therefore also for the prevention of congenital infection.

The vaccine provided by the invention is also able to prevent HCMV congenital infection in newborns. The vaccine is to be administered to the seronegative women or the seronegative young mothers as an effective amount of a vaccine composition containing as antigen the gB of HCMV in an O/W emulsion (oil in water emulsion) before the conception of said newborn.

The term "prevention of HCMV congenital infection in a newborn" means that the newborn is free of HCMV infection at birth. This results from the absence of transmission of any HCMV from the pregnant child-bearing age woman to the fetus throughout pregnancy.

It was indeed observed within the frame of the clinical study that among the 112 live newborns born to mothers who were given the placebo, 5 were HCMV infected. By contrast, none of the 93 live newborns born to mothers who where vaccinated were HCMV infected. These results show that the vaccine of the invention not only prevents HCMV infection in child bearing age women but also prevents to a greater extent HCMV congenital infection in newborns.

The vaccine composition used for the prevention of HCMV congenital infection in newborns, as it is used for the prevention of HCMV infection in HCMV seronegative subjects, can contain one or several other antigens of HCMV formulated in an O/W emulsion or only the gB antigen as HCMV antigen formulated in an O/W emulsion since it has been shown that gB alone is enough to prevent HCMV congenital infection in newborns.

The HCMV gB component of the vaccine composition useful for the purpose of the invention is a full length gB polypeptide or a gB-derived polypeptide that induces neutralizing antibodies.

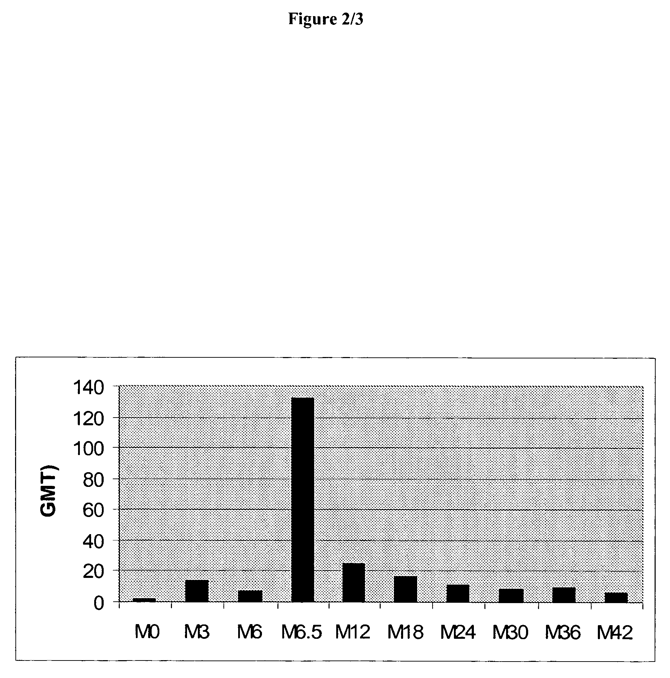

gB is encoded by the UL55 gene of HCMV genome. The size of the native form of gB (or gp130) depends on the size of the open reading frame (ORF), which may vary a little according to the strain. For example, The ORF of AD169 strain, which is 2717 bp long, encodes a full length gB of 906 amino acids whereas the ORF of Towne strain encodes a full length gB of 907 amino acids. The protein sequences of these two strains are described in US 2002/0102562 (figure 2). The native form of gB contains an amino acid signal sequence that is normally 23 or 24 amino acid long, followed by an extracellular domain containing an endoproteolytic cleavage site between residues arginine 460 and serine 461, by a transmembrane domain and by an intracellular domain. Usually, the full length gB is depleted of the amino acid signal sequence as a consequence of posttranslational mechanisms that occur in cells. It will be well understood that suitable full length gB for the purpose of the invention encompasses both the full length gB of HCMV strains Towne and AD 169, as well as other equivalent strains. Several antigenic domains inducing neutralizing antibodies have been described. Notably, it includes the domain that is located between amino acid residues 461 and 680 of gp 130, this domain being subdivided into two discontinuous domains, the domain between residues 461 and 619 and the domain between residues 620 and 680 (US 5,547,834). It also includes the antigenic domain 1 (AD-1) located between amino acid residues 552 and 635 or the antigenic domain 2 (AD-2) located between amino acid residues 50 and 77 (Journal of General Virology (1999), 80, 2183-2191; Journal of Virology (2005), 79, 4066-4079) or between amino acid residues 27 and 84 (Journal of Virology (2003), 305, 201-209). Consequently, a polypeptide that comprises in its amino acid sequence a sequence homologous to one or several of the above cited antigenic domains is also suitable for the purpose of the invention. The term "a sequence homologous to" is intended to mean an amino acid sequence in which there is at least 80% identity with the amino acid sequence of the antigenic domain being considered of the native gB originating from the Towne or AD169 strain (which are described in US 2002/0102562). Typically, the sequence homology is based on a sequence identity of at least 90% and, even more specifically, the sequence homology is complete (sequence identity of 100%).

Among the gB-derived peptides or polypeptides that are suitable for the object of the invention is gp 55 as described in US 5,547,834. It is derived from the cleavage of gB at the endoproteolytic cleavage site; its amino acid sequence corresponds to that which is between serine residue 461 and the C-terminal end. Truncated forms of gp 55 can also be used, such as a gp 55 depleted of all or part of the transmembrane sequence and of all or part of the intracellular C-terminal domain (for example, a peptide having a sequence homologous to the amino acid sequence of the native gB between residues 461 and 646) or a gp 55 depleted of all or part of the intracellular C-terminal domain (for example, a peptide having a sequence homologous to the amino acid sequence of the native gB between residues 461 and 680). Such truncated forms of gp 55 are also described in US 5,547,834.

It is also possible to use a mutated form of the full length gB that carries one or more mutations at the endoproteolytic cleavage site such that the latter is made ineffectual. The mutation(s) is (are) located between residues 457 and 460 of the sequence of gp130 and, more particularly, are located at arginine 460 and/or lysine 459 and/or arginine 457. In this aspect, the mutated form of the full length gB carries the entire extracellular domain with all the domains that are targets for neutralizing antibodies. Such mutated forms can be secondarily depleted of all or part of the transmembrane sequence and/or of all or part of the intracellular C-terminal domain. Such gB-derivatives are preferred in so far as substantially all the domains that are targets for neutralizing antibodies are conserved.

Therefore, in a preferred aspect of the invention the HCMV gB comprises one or several mutations on the cleavage site, and preferably the HCMV gB is in addition selected from among the group of a full length HCMV gB, a full length HCMV gB lacking substantially all the transmembrane domain, a full length HCMV gB lacking substantially all the intracellular domain, and a full length HCMV gB polypeptide lacking substantially both the transmembrane domain and the intracellular domain.

The term "lacking substantially all the intracellular domain" or "lacking substantially all the transmembrane domain" means that at least 80% of the amino acid sequence corresponding to the said domain is deleted

A HCMV gB antigen that is particularly suitable for the subject of the invention is a truncated form of the full length gB depleted of all or part of the C-terminal domain and/or depleted of all or part of the transmembrane sequence and in which the cleavage site is ineffectual. A truncated form of gB that is particularly preferred corresponds to that which is described in US 6,100,064, called gBdTM; it carries three mutations at the cleavage site and a deletion in the transmembrane region between amino acid residues valine 677 and arginine 752, such that the extracellular domain is directly connected to the cytoplasmic domain. Such gB-derived polypeptide is easier to purify as it is produced by recombinant cells expressing this product under a secreted form. The resulting form is an 807 amino acid long polypeptide deleted of its signal sequence and of its transmembrane region when it is derived from the gB Towne strain.

The HCMV gB protein or the peptides or polypeptides derived therefrom are usually obtained by recombinant DNA techniques and purified according to methods well known to those skilled in the art. The methods described in US 6,100,064 and in US 2002/0102562, can in particular be used. To increase their immunogenicity, they can secondarily be conjugated to a carrier protein or fused to other proteins, in particular to particle-forming proteins such as the hepatitis B surface antigen. An example of fusion protein that is suitable for the purpose of the invention is given in WO 95/31555, wherein the N terminal part of a portion of HCMV gB is fused to a C terminus of a portion of HSV gD protein. Preferably, the portions of HCMV gB and HSV gD lack substantially the transmembrane domains, and, furthermore, the portion of HCMV gB comprises one or several mutations on the cleavage site. Examples of suitable structures are designated HCMV gB 685* and HCMVgB685**.

The O/W emulsion suitable for the subject of the invention comprises at least a metabolizable oil, an emulsifying agent, and an aqueous solution wherein the oil and the emulsifying agent are present in the form of an oil-in water emulsion having oil droplets substantially all of which are less than 1 micron diameter, more typically smaller than 250nm. Furthermore, the emulsion shall be "pharmaceutically acceptable", which means that the material may be administered to an individual without causing any excessively undesirable biological effects in the individual or interacting in an excessively deleterious manner with any of the components of the emulsion. Other components that are present in the O/W emulsion, among them the gB antigen and various supplements, may independently be, for example, dissolved or dispersed within the oil phase (s) of the emulsion (including separate populations of oil droplets), dissolved or dispersed within the aqueous phase of the emulsion and/or disposed at the interfaces between aqueous and oil phases of the emulsion.

The gB-containing O/W emulsion ready to be administered comprises the gB antigen, a metabolizable oil (wherein the volume of oil represents 0.5 to 20% of the total volume of the emulsion (v/v), in particular 1 to 10% (v/v) and more particularly 1 to 5% (v/v)), an aqueous solution (wherein the volume of the aqueous solution represents 80 to 99.5% of the total volume (v/v), in particular 90 to 99 % (v/v)) and one or several emulsifying agent(s) (wherein the total amount of the emulsifying agent represents 0.001 to 5 % of the total amount of the emulsion (w/w), in particular 0.001 to 2% (w/w), and more particularly 0.01 to 2% (w/w)).

The metabolizable oil is commonly one having about 6 to about 30 carbon atoms including, but not limited to, alkanes, alkenes, alkynes, and their corresponding acids and alcohols, the ethers and esters thereof, and mixtures thereof. The oil can be essentially any plant oil, fish oil, animal oil or synthetically prepared oil that can be metabolized by the body of the human subject to which the emulsion compositions will be administered and that is not substantially toxic to the subject.

For example, the oil component of this invention can be any long chain alkane, alkene, or alkyne, or an acid or alcohol derivative thereof, for example, as the free acid, its salt or an ester thereof, such as a mono-, or di-or tri-esters, for instance, triglycerides, esters of 1,2-propanediol or similar poly-hydroxy alcohols. Alcohols can be acylated employing amino-or poly-functional acid, for example acetic acid, propanoic acid, citric acid or the like. Ethers derived from long chain alcohols that are oils and meet the criteria set forth herein can also be used.

The individual alkane, alkene, or alkyne moiety and its acid or alcohol derivatives will generally have about 6 to about 30 carbon atoms. The moiety can have a straight or branched chain structure. It can be fully saturated or have one or more double or triple bonds. Where mono or poly ester-or ether-based oils are employed, the limitation of about 6 to about 30 carbons applies to the individual fatty acid or fatty alcohol moieties, not the total carbon count.

The metabolizable oil is generally selected from animal oils (including fish oils) and plant oils. It can be an unsaturated hydrocarbon having from 20-40 carbons, or a branched, polyunsaturated hydrocarbon having from 20-40 carbon atoms, for example, terpenoids. An unsaturated terpenoid known as squalene, 2,6,10,15,19,23-hexamethyl-2,6,10,14,18,22-tetracosahexaene and its saturated analog, squalane, are often preferred. Fish oils, including squalene and squalane, are readily available from commercial sources or may be obtained by methods known in the art. Another oil commonly used is tocopherol. Where a composition includes a tocopherol, any of the α, β, γ, δ, ε or ξ tocopherols can be used but α-tocopherols are preferred.
A substantial number of suitable emulsifying agents (also referred to herein as surfactants, detergents and so forth) are used in the pharmaceutical sciences, many of which are useful in the composition of the emulsion of the present invention, so long as they are sufficiently non-toxic. These include naturally derived materials such as gums from trees, vegetable protein, sugar-based polymers such as alginates and cellulose, and the like. Certain oxypolymers or polymers having a hydroxide or other hydrophilic substituent on the carbon backbone have surfactant activity, for example, povidone, polyvinyl alcohol, and glycol ether-based mono-and poly-functional compounds. Long chain fatty-acid-derived compounds form another substantial group of emulsifying agents that could be used in this invention.

Specific examples of suitable emulsifying agents that can be used in accordance with the present invention include the following:
(1) Water-soluble soaps, such as the sodium, potassium, ammonium and alkanol-ammonium salts of higher fatty acids (C10-C22), and, particularly sodium and potassium tallow and coconut soaps.
(2) Anionic synthetic non-soap detergents, which can be represented by the water-soluble salts of organic sulfuric acid reaction products having in their molecular structure an alkyl radical containing from about 8 to 22 carbon atoms and a radical selected from the group consisting of sulfonic acid and sulfuric acid ester radicals.
(3) Nonionic synthetic detergents made by the condensation of alkylene oxide groups with an organic hydrophobic compound. Typical hydrophobic groups include condensation products of propylene oxide with propylene glycol, alkyl phenols, the condensation product of propylene oxide and ethylene diamine, aliphatic alcohols having 8 to 22 carbon atoms, and amides of fatty acids.
(4) Nonionic detergents, such as amine oxides, phosphine oxides and sulfoxides, having semipolar characteristics.
(5) Long chain sulfoxides, including those corresponding to the formula R1-SO-R2 wherein R1 and R2 are substituted or unsubstituted alkyl radicals, the former containing from about 10 to about 28 carbon atoms, whereas R2 contains from I to 3 carbon atoms.
(6) Ampholytic synthetic detergents, such as sodium 3-dodecylaminopropionate and sodium 3- dodecylaminopropane sulfonate.
(7) Zwitterionic synthetic detergents, such as 3- (N, N- dimethyl-N-hexadecylammonio) propane-1-sulfonate and 3- (N, N-dimethyl-N-hexadecylammonio) -2-hydroxy propane-1-sulfonate.

The following types of emulsifying agents, which are not necessarily exclusive of those in the prior paragraph, can also be used in the emulsion compositions of the present invention: (a) soaps (i.e., alkali salts) of fatty acids, rosin acids, and tall oil; (b) alkylarene sulfonates; (c) alkyl sulfates, including surfactants with both branched-chain and straight-chain hydrophobic groups, as well as primary and secondary sulfate groups; (d) sulfates and sulfonates containing an intermediate linkage between the hydrophobic and hydrophilic groups, such as the fatty acylated methyl taurides and the sulfated fatty monoglycerides; (e) long-chain acid esters of polyethylene glycol, especially the tall oil esters; (f) polyethylene glycol ethers of alkylphenols; (g) polyethylene glycol ethers of long-chain alcohols and mercaptans; and (h) fatty acyl diethanol amides.

There are a number of emulsifying agents specifically designed for and commonly used in biological situations. For example, a number of biological detergents (surfactants) are listed as such by Sigma Chemical. Such surfactants are divided into four basic types: anionic, cationic, zwitterionic, and nonionic.

Examples of anionic detergents include alginic acid, caprylic acid, cholic acid, 1-decanesulfonic acid, deoxycholic acid, 1-dodecanesulfonic acid, N-lauroylsarcosine, and taurocholic acid.

Cationic detergents include dodecyltrimethylammonium bromide, benzalkonium chloride, benzyldimethylhexadecyl ammonium chloride, cetylpyridinium chloride, methylbenzethonium chloride, and 4-picoline dodecyl sulfate.

Examples of zwitterionic detergents include 3-[(3-cholamidopropyl)-dimethylammonio]-l-propanesulfonate (commonly abbreviated CHAPS), 3-[(cholamidopropyl) dimethylammoniol-2-hydroxy-l-propanesulfonate (commonly abbreviated CHAPSO), N-dodecyl-N,N-dimethyl-3-ammonio-1-propanesulfonate, phosphatidylcholine and lyso-alpha-phosphatidylcholine.

Examples of nonionic detergents includedecanoyl-N-methylglucamide, diethylene glycol monopentyl ether, n-dodecyl beta-D-glucopyranoside, poloxamers, ethylene oxide condensates of fatty alcohols (e.g., those sold under the trade name Lubrol), polyoxyethylene ethers of fatty acids (particularly C12-C20 fatty acids), polyoxyethylene sorbitan fatty acid esters (e.g., sold under the trade name Tween®), and sorbitan fatty acid esters (e.g., sold under the trade name Span®).

A particularly useful group of surfactants are the sorbitan-based non-ionic surfactants. These surfactants are typically prepared by dehydration of sorbitol to give 1,4-sorbitan, which is then reacted with one or more equivalents of a fatty acid. The fatty- acid-substituted moiety may be further reacted with ethylene oxide to give a second group of surfactants.

The fatty-acid-substituted sorbitan surfactants are typically made by reacting 1,4-sorbitan with a fatty acid such as lauric acid, palmitic acid, stearic acid, oleic acid, or a similar long chain fatty acid to give the 1,4-sorbitan mono-ester, 1,4-sorbitan sesquiester or 1,4-sorbitan triester. The common names for some of these surfactants include, for example, sorbitan monolaurate, sorbitan monopalmitate, sorbitan monostearate, sorbitan monooleate, sorbitan sesquioleate, and sorbitan trioleate. These surfactants are commercially available under the names SPAN^{®} or ARLACEL^{®}. SPAN^{®} and ARLACEL^{®} surfactants are lipophilic and are generally soluble or dispersible in oil. They are also soluble in most organic solvents. In water they are generally insoluble but dispersible. Generally these surfactants will have a hydrophilic-lipophilic balance (HLB) number between 1.8 and 8.6. Such surfactants can be readily made by means known in the art or are commercially available.

A related group of surfactants comprises polyoxyethylene sorbitan monoesters and polyoxyethylene sorbitan triesters. These materials are typically prepared by addition of ethylene oxide to a 1,4-sorbitan monoester or triester. The addition of polyoxyethylene converts the lipophilic sorbitan mono-or triester surfactant into a hydrophilic surfactant generally soluble or dispersible in water and soluble to varying degrees in organic liquids. The TWEEN^{®} surfactants may be combined, for example, with a related sorbitan monoester or triester surfactant to promote emulsion stability. TWEEN^{®} surfactants generally have a HLB value falling between 9.6 and 16.7. TWEEN^{®} surfactants are commercially available from a number of manufacturers, for example ICI America's Inc., Wilmington, Del. under the registered mark ATLAS^{®} surfactants.

Another group of non-ionic surfactants that could be used alone or in conjunction with SPAN^{®}, ARLACEL^{®} and/or TWEEN^{®} surfactants are the polyoxyethylene fatty acids made by the reaction of ethylene oxide with a long-chain fatty acid. The most commonly available surfactant of this type is solid under the name MYRJ^{®} and is a polyoxyethylene derivative of stearic acid. MYRJ^{®} surfactants are hydrophilic and soluble or dispersible in water, like TWEEN^{®} surfactants. The MYRJ^{®} surfactants may be blended, for example, with TWEEN^{®}surfactants or with TWEEN^{®}/SPAN^{®} or with ARLACEL^{®} surfactant mixtures for use in forming emulsions. MYRJ^{®)} surfactants can be made by methods known in the art or are available commercially from ICI America's Inc.

Another group of polyoxyethylene based non-ionic surfactants are the polyoxyethylene fatty acid ethers derived from lauryl, acetyl, stearyl and oleyl alcohols. These materials are typically prepared as above by addition of ethylene oxide to a fatty alcohol. The commercial name for these surfactants is BRIJ^{®}; BRIJ^{®} surfactants may be hydrophilic or lipophilic depending on the size of the polyoxyethylene moiety in the surfactant. While the preparation of these compounds is available from the art, they are also readily available from such commercial sources as ICI America's Inc.

Other non-ionic surfactants that may be used in the practice of this invention are, for example: polyoxyethylenes, polyol fatty acid esters, polyoxyethylene ethers, polyoxypropylene fatty ethers, bee's wax derivatives containing polyoxyethylene, polyoxyethylene lanolin derivatives, polyoxyethylene fatty glycerides, glycerol fatty acid esters or other polyoxyethylene acid alcohols or ether derivatives of long-chain fatty acids of 12-22 carbon atoms.

As noted above, in certain embodiments, two or more surfactants can be combined in the emulsion compositions of the present invention. For instance, the O/W emulsion can comprise a hydrophilic emulsifying agent having an HLB value ranging from 1-9 and a lipophilic emulsifying agent having an HLB value ranging from 10-18. As a specific example, a sorbitan fatty acid ester can be combined with a polyoxyethylene sorbitan fatty acid ester (see Table below, which lists several of these emulsifiers, along with their associated HLB values).

| Emulsifier | HLB |
|---|---|
| Sorbitan trioleate (Span^{®} 85) | 1.8 |
| Sorbitan tristearate (Span^{®}65) | 2.1 |
| Sorbitan sesquioleate (Arlacel^{®} 83) | 3.7 |
| Sorbitan monooleate (Span^{®} 80) | 4.3 |
| Sorbitanmonostearate (Span^{®} 60) | 4.7 |
| Sorbitanmonopalmitate (Span^{®} 40) | 6.7 |
| Sorbitan monolaurate (Span^{®} 20) | 8.6 |
| Polyoxyethylene sorbitan tristearate (Tween^{®} 65) | 10.5 |
| Polyoxyethylene sorbitan trioleate (Tween^{®} 85) | 11.0 |
| Polysorbate 60 (Tween^{®} 60) | 14.9 |
| Polysorbate 80 (Tween^{®} 80) | 15.0 |
| Polysorbate 40 (Tween^{®} 40) | 15.6 |
| Polysorbate 20 (Tween^{®} 20) | 16.7 |

The aqueous solution of the O/W emulsion of the invention is buffered saline or, in other embodiments, unadulterated water. Because the composition of the invention is intended for parenteral administration, it is preferable to make up final buffered solutions used as vaccines so that the tonicity, i.e., osmolality, is essentially the same as normal physiological fluids in order to prevent post-administration swelling or rapid absorption of the composition because of differential ion concentrations between the composition and physiological fluids. It is also preferable to buffer the saline in order to maintain a pH compatible with normal physiological conditions. Also, in certain instances, it may be necessary to maintain the pH at a particular level in order to insure the stability of the gB antigen and eventually other HCMV antigens if they are present in the O/W emulsion.

Any physiologically acceptable buffer may be used herein, but phosphate buffers are preferred. Other acceptable buffers such as acetate, tris, bicarbonate, carbonate, citrate or the like may be used as substitutes for phosphate buffers. The pH of the aqueous component will preferably be between 6.0 and 8.0.

Usually the O/W emulsion comprises at least one non-ionic surfactant, such as fatty acid esters and/or fatty acid esters comprising a polyoxyethylene moiety. Mention is made, for example, of sorbitan derivatives such as sorbitan fatty acid monoesters, sorbitan fatty acid sesquiesters, sorbitan fatty acid triesters, polyoxyethylene sorbitan fatty acid monoesters and polyoxyethylene sorbitan fatty acid triesters.

Emulsifying agents that are often used alone or in combination in the composition of the emulsion include Tween 80 (polyoxyethylene sorbitan monooleate), Span 85 (sorbitan trioleate), lecithin and triton X-100 (t-ocylphenoxypolyethoxyethanol). Where there are two or more surfactants, one surfactant can have, for example, an HLB value ranging from 1 to 9, while the other surfactant can have an HLB value ranging from 10 to 20.

Examples of specific O/W emulsions within the scope of the invention include but are not limited to:
- An emulsion of squalene, tocopherol, and Tween 80. The aqueous solution may be a phosphate buffered saline. It may also include Span 85 (e.g. at 1%) and/or lecithin. These emulsions may have from 2 to 10% squalene, from 2 to 10% tocopherol and from 0.3 to 3% Tween 80, and the weight ratio of squalene/ tocopherol is preferably ≤1 as this provides a more stable emulsion. Squalene and Tween 80 may be present at a volume ratio of about 5:2. One such emulsion can be made by dissolving Tween 80 in PBS to give a 2% solution, then mixing 90 ml of this solution with a mixture of (5 g of DL-α tocopherol and 5 ml squalene), then microfluidising the mixture as mentioned below.
- An emulsion of squalene, a tocopherol, and a Triton detergent (e.g., Triton X-100).
- An emulsion comprising a polysorbate (e.g. polysorbate 80), a Triton detergent (e.g., Triton X-100) and a tocopherol (e.g., α-tocopherol succinate). The emulsion may include these three components at a mass ratio of about 75:11:10 (e.g. 750 µg/ml polysorbate 80, 110 µg/ml Triton X-100 and 100 µg/ml α-tocopherol succinate). The emulsion may also include squalene.
- An emulsion of squalane, polysorbate 80 and poloxamer 401 ("Pluronic(TM) L121"). The emulsion can be formulated in phosphate buffered saline, pH 7.4. A particular composition of this emulsion comprises 5% squalane, 1.25% Pluronic L121 and 0.2% polysorbate 80.
- An emulsion of squalene, phosphatidylcholine, poloxamer 188, glycerol and α-tocopherol.

A more particular O/W emulsion, called MF-59 like emulsion, comprises 3 to 6% squalene (v/v), and a mixture of polyoxyethylene sorbitan monooleate and sorbitan triooleate as emulsifying agents, wherein the total amount of emulsifying agents represents 0.5 to 1.5% of the total amount of the emulsion (w/w), and an aqueous solvent. Usually, the aqueous solvent of the MF-59 like emulsion is a buffer solution such as citrate buffer.

An example of particularly suitable composition of MF-59 like emulsion is given below:

| Component | Quantity (per vial containing 0.7 ml) |
|---|---|
| Squalene | 27.3 mg |
| Polyoxyethylene sorbitan monooleate (Tween 80) | 3.29 mg |
| Sorbitan trioleate (Span 85) | 3.29 mg |
| Sodium citrate, dihydrate | 1.855 mg |
| Citric acid, monohydrate | 0.119 mg |
| Water for injection | Qs to 0.7 ml |

The O/W emulsion of the present invention is generally prepared by microfluidisation techniques. Commercial emulsifiers can be used, which operate by the principle of high shear forces developed by forcing fluids through small apertures under high pressure. Examples of commercial emulsifiers include, without limitation, Model110Y microfluidizer (Microfluidics, Newton, Mass.), Gaulin Model 30CD (Gaulin, Inc., Everett, Mass.), and Rainnie Minilab Type 8.30H (Miro Atomizer Food and Dairy, Inc., Hudson, Wis.). The appropriate pressure for use with an individual emulsion is readily determined by one of skill in the art. Further information regarding the preparation of O/W emulsions can be found in US 6,299,884. Substantially all oil droplets in the O/W emulsion obtained are usually less than 1 micron in diameter, in particular less than 0.8 microns in diameter, and more particularly less than 0.5 microns in diameter.

By "substantially all" is meant at least about 80% (by volume of the oil droplets), preferably at least about 90%, more preferably at least about 95% or even at least 98%. The particle size distribution is typically Gaussian, so that the average diameter is smaller than the stated limits.

Droplet size varies with the surfactant and oil used, as well as other components present, if any, at the time of emulsification (e.g., optional supplemental compounds discussed below).

The size of the oil droplets is also modified by changing the ratio of emulsifying agent to oil (increasing the ratio typically decreases droplet size), by operating pressure (increasing operating pressure typically decreases droplet size), and by operating temperature (increasing temperature typically decreases droplet size).

Droplet size is controlled by use of sizing instruments, such as the commercial Sub-Micron Particle Analyzer (Model N4MD) manufactured by the Coulter Corporation, or the laser diffraction particle size analyser such as the Beckman Coulter device of the LS range (in particular the LS230).

Usually, for the so called MF59-like emulsions, substantially all oil droplets are less than 180 nm.

The vaccine composition that comprises the gB antigen in the MF-59-like emulsion may be then prepared by mixing, for instance, one volume of the MF-59-like emulsion to one volume of an aqueous solution of the gB antigen.

An easier process that is reproducible and completely reliable may be applied to thermoreversible O/W emulsions that comprise as components, squalene, an aqueous solvent, a hydrophilic non ionic polyoxyethylene alkyl ether and an hydrophobic non ionic surfactant. The process uses the property of such emulsions to change from the state of an O/W emulsion to the state of a W/O emulsion when they are heated at a temperature at least equal to the phase inversion temperature. The embodiments of the process are well described in WO 2007/006939, and are especially suitable for the preparation of thermoreversible O/W emulsions that contain usually from 5 to 45% squalene (w/w), from 0.9 to 9% polyoxyethylene alkyl ether (w/w) and from 0.7 to 7% hydrophobic non ionic surfactant (w/w) such as sorbitan ester or mannide ester based surfactants with a HLB less than 9. Preferably, the polyoxyethylene alkyl ether is chosen from the group consisting of ceteareth-12 (sold under the name Eumulgin® B1), ceteareth-20 (Eumulgin® B2), steareth-21 (Eumulgin® S21), ceteth-20 (Simulsol® 58 or Brij® 58), ceteth-10 (Brij® 56), steareth-10 (Brij® 76), steareth-20 (Brij® 78), oleth-10 (Brij® 96 or Brij® 97) and oleth-20 (Brij® 98 or Brij® 99). The number attributed to each chemical name corresponds to the number of ethylene oxide units in the chemical formula. In a particular aspect, the polyoxyethylene alkyl ether is BRIJ® 56 or polyoxyethylene (12) cetostearyl ether, provided by the company Cognis under the name Eumulgin^{™} B1. Among the sorbitan ester and mannide ester based surfactants with a HLB less than 9 that are particularly suitable, mention may be made of the sorbitan monooleate sold under the name Dehymuls SMO^{™} or Span®80. Among the mannide ester-based surfactants, mention may be made of the mannide monooleate sold by the company Sigma, or by the company Seppic under the name Montanide 80^{™}. Mannitol in a range of 1 to 10% (w/w) may be used as supplement of such thermoreversible O/W emulsions when it is desirable to decrease the phase inversion temperature. By virtue of the preparation process used, the droplets of oil of the thermoreversible emulsion all have virtually the same size, which is very small; in fact, when the size distribution curve is represented for the drops obtained, it is noted that a monodisperse emulsion is obtained, with a very tight Gaussian-type distribution, centered around a single low value, generally around 80-90nm.

The O/W emulsion according to the invention may comprise supplemental components that can be added at the time of preparing the O/W emulsion or added once the O/W emulsion is prepared. It may be a lyophilization substrate comprising an alditol, a sugar and an alkylpolyglycoside. A lyophilization substrate normally used comprises mannitol, sucrose or dodecyl maltoside and allows the O/W emulsion to be conserved in the form of lyophilizate. It may be components that improve the stability of the O/W emulsion such as antioxidant like α tocopherol and/or that improve the stability of the gB antigen.

The O/W emulsion may be supplemented with immunological adjuvants like aluminium salts or muramyl peptides (e.g., N-acetyl-muramyl-L- threonyl-D-isoglutamine (thr-MDP), N-acteyl-normuramyl-L-alanyl-D-isogluatme (norMDP), N-acetylmuramyl-L-alanyl-D-isogluatminyl-L-alanine-2-(1'-2'-dipalmitoyl-sn-glycero-3-hydroxyphosphoryloxy)-ethylamine (MTP-PE), etc.) that may be used to enhance the effectiveness of the vaccine composition.

In particular, it may be desirable to improve more the TH-1 component of the immune response and to incorporate a TH-1 adjuvant in the vaccine composition according to the invention. TH-1 immune response is usually considered as the cellular component of the immune response and involves cytotoxic T lymphocytes, and natural killer cell responses. In mice, TH1-type response is often characterized by an increased production of antibodies of the IgG2a isotype, whilst in the human it corresponds to an increased production of IgG 1 type antibodies. On the other hand, TH2-type immune response in mice is characterized by the generation of a range of immunoglobulin isotypes, and in particular the generation of IgG1 isotype. It can be considered that cytokines are the driving force behind the development of these two types of immune responses. High levels of TH1-type cytokines tend to favor the induction of cell mediated immune responses to the given antigen, whilst high levels of TH2-type cytokines tend to favor the induction of humoral immune responses to the antigen. The distinction between TH1 and TH2-type immune response is not absolute. In reality an individual will support an immune response which is described as being predominantly TH1 or predominantly TH2. However, it is often convenient to consider the families of cytokines in terms of that described in murine CD4 positive T cell clones by Mosmann and Coffman (Mosmann, T. R. and Coffman, R. L. (1989) TH1 and TH2 cells: different patterns of lymphokine secretion lead to different functional properties. Annual Review of Immunology, 7, p 145-173). Traditionally, TH1-type response is associated with the production of the INF-γ by T-lymphocytes. There are other cytokines often directly associated with the induction of TH1-type immune response that are not produced by T-cells, like IL-12. In contrast, TH2-type response is associated with the secretion of IL4, IL-5, IL-6, IL-10 and TNFβ. It is known that certain vaccine adjuvants are particularly suited to the stimulation of either TH1 or TH2-type cytokine responses. Traditionally, one of the preferred indicators of the TH1:TH2 balance of the immune response after a vaccination includes direct measurement of the production of TH1 or TH2 cytokines by T lymphocytes in vitro after re-stimulation with antigen, and/or (at least in mice) the measurement of the IgG1:IgG2a ratio of antigen specific antibody responses. Thus, a TH1-type adjuvant is one which stimulates T-cell populations to produce high levels of TH1-type cytokines when re-stimulated with antigen in vitro, or induces antigen specific immunoglobulin responses associated with TH1-type isotype.

Adjuvants which are capable of preferential stimulation of the TH1-type response are described in WO 94/00153 and WO 95/17209. 3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem, Montana. A preferred form of 3 De-O-acylated monophosphoryl lipid A is disclosed in EP 0 689 454. Usually, the particles of 3D-MPL are small enough to be sterile filtered through a 0.22 micron membrane (as described in EP 0 689 454). 3D-MPL is present in the range of 10 µg-100 µg, more particularly within the range of 25-50 µg whereas the antigen is usually present in a range 5-100 µg per vaccine dose. A 3D-MPL derivative, named RC-529, is described in US 6,113,918 and represents an alternative to 3D-MPL as TH-1 adjuvant.

Another useful class of TH-1 adjuvant is represented by TLR4 agonist represented by the chemical molecules of formula I, II, III or IV: 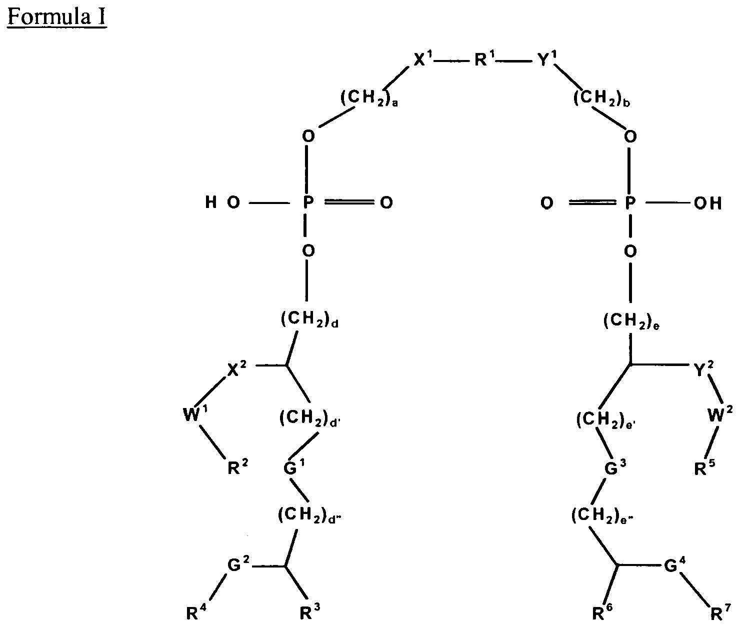 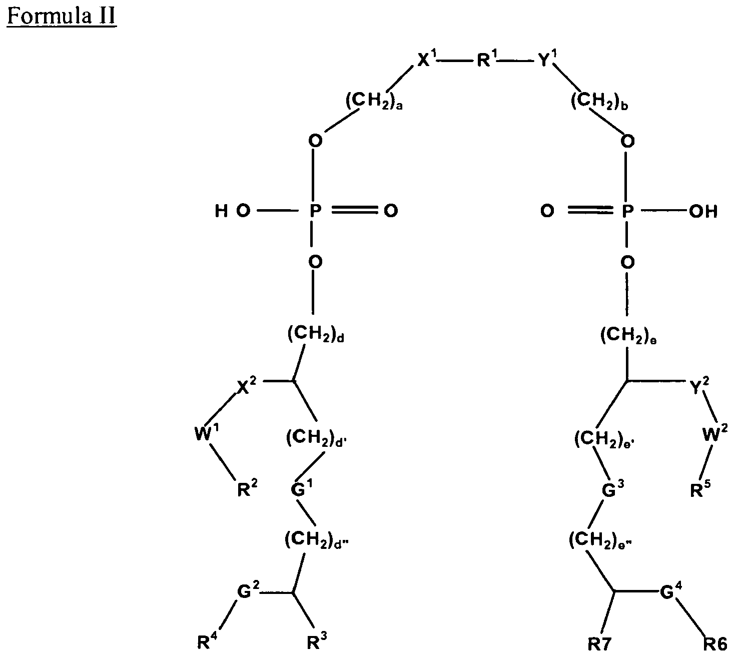 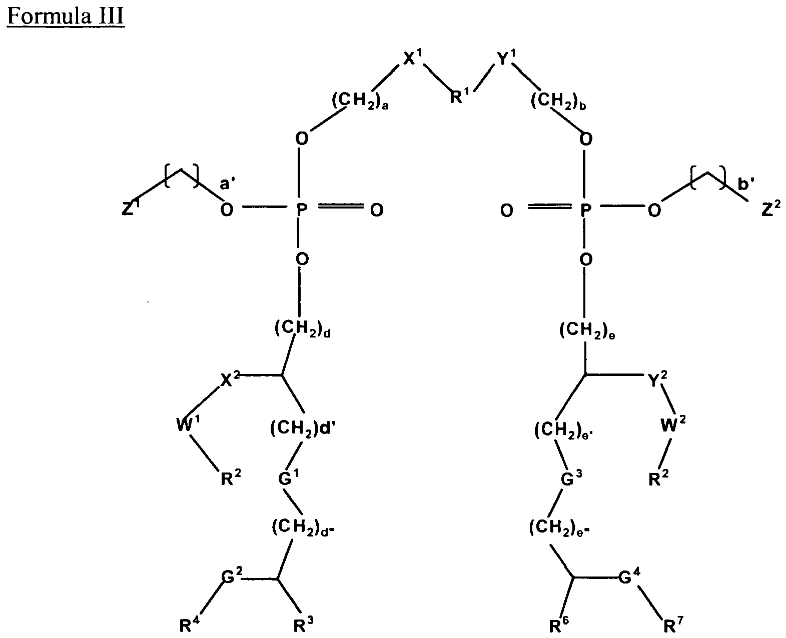 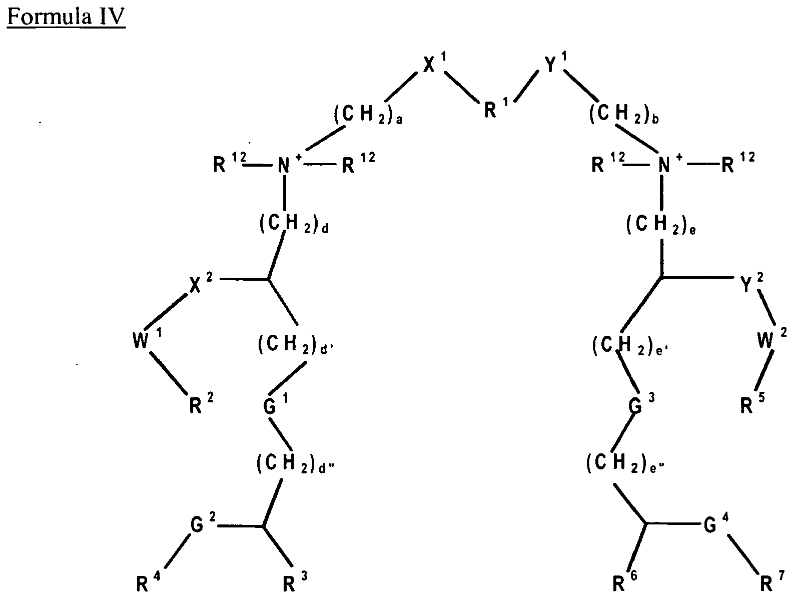 in which, for each of the formulae I, II, III or IV, R¹ is selected from the group consisting of:
a) C(O);
b) C(O)-(C₁-C₁₄ alkyl)-C(O), in which said C₁-C₁₄ alkyl is optionally substituted with a hydroxy, aC₁-C₅ alkoxy, a C₁-C₅ alkylenedioxy, a C₁-C₅ alkylamino or a C₁-C₅ alkyl-aryl, in which said aryl moiety of said C₁-C₅ alkyl-aryl is optionally substituted with a C₁-C₅ alkoxy, a C₁-C₅ alkylamino, a C₁-C₅ alkoxy-amino, a C₁-C₅ alkylamino-C₁-C₅ alkoxy, -O-C₁-C₅ Alkylamino-C₁-C₅ alkoxy, -O-C₁-C₅ alkylamino-C(O)-C₁-C₅ alkyl-C(O)OH, or -O-C₁-C₅ alkylamino-C(O)-C₁-C₅ alkyl-C(O)-C₁-C₅alkyl;
c) an alkyl comprising a C₂-C₁₅ straight or branched chain, optionally substituted with a hydroxyl or an alkoxy; and
d) -C(O)-C₆-C₁₂ arylene-C(O)- in which said arylene is optionally substituted with a hydroxy, a halogen, a nitro or an amino;
   a and b are independently 0, 1, 2, 3 or 4;
   d, d', d", e, e' and e" are independently an integer from 0 to 4;
   X¹, X², Y¹ and Y² are independently selected from the group consisting of a chemical bond, an oxygen, NH and N(C(O) C₁-C₄ alkyl), and N(C₁-C₄ alkyl);
   W¹ and W² are independently selected from the group consisting of carbonyl, methylene, sulfone and sulfoxide;
   R² and R⁵ are independently selected from the group consisting of:
      a) C₂ to C₂₀ straight chain or branched chain alkyl, which is optionally substituted with an oxo, a hydroxy or an alkoxy;
      b) C₂ to C₂₀ straight chain or branched chain alkenyl or dienyl, which is optionally substituted with an oxo, a hydroxy or an alkoxy;
      c) C₂ to C₂₀ straight chain or branched chain alkoxy, which is optionally substituted with an oxo, a hydroxy or an alkoxy;
      d) -NH-C₂ to C₂₀ straight chain or branched chain alkyl, in which said alkyl group is optionally substituted with an oxo, a hydroxy or an alkoxy; and 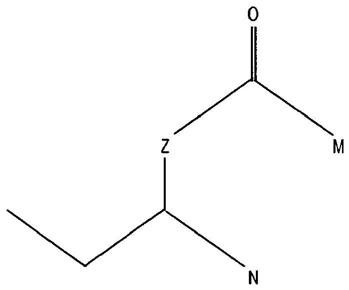 in which Z is selected from the group consisting of O and NH, and M and N are independently selected from the group consisting of C₂ to C₂₀ straight chain or branched chain alkyl, alkenyl, alkoxy, acyloxy, alkylamino and acylamino;
   R³ and R⁶ are independently selected from the group consisting of C₂ to C₂₀ straight chain or branched chain alkyl or alkenyl, optionally substituted with an oxo or a fluoro;
   R⁴ and R⁷ are independently selected from the group consisting of C(O)-C₂ to C₂₀ straight chain or branched chain alkyl or alkenyl; C₂ to C₂₀ straight chain or branched alkyl; C₂ to C₂₀ straight chain or branched chain alkoxy; C₂ to C₂₀ straight chain or branched chain alkenyl; in which said alkyl, alkenyl or alkoxy groups can be independently and optionally substituted with a hydroxy, a fluoro or a C₁-C₅ alkoxy;
   G¹, G², G³ and G⁴ are independently selected from the group consisting of oxygen, methylene, amino, thiol, -C(O)NH-, -NHC(O)-, and -N(C(O) C₁-C₄ alkyl)-;
   or G²R⁴ or G⁴R⁷ can together be a hydrogen atom or a hydroxy;
   and in which, for formula III:
      a' and b' are independently 2, 3, 4, 5, 6, 7 or 8, preferably 2;
      Z¹ is selected from the group consisting of -OP(O)(OH)₂, -P(O)(OH)₂, -OP(O)(OR⁸)(OH) where R⁸ is a C₁-C₄ alkyl chain, -OS(O)₂OH, -S(O)₂OH,-CO₂H,
         -OB(OH)₂, -OH, -CH₃, -NH₂ and -N(R⁹)₂ where each R⁹ is independently a C₁-C₄ alkyl chain;
      Z² is -OP(O)(OH)₂, -P(O)(OH)₂, -OP(O)(OR¹⁰)(OH) where R¹⁰ is a C₁-C₄ alkyl chain, -OS(O)₂OH, -S(O)₂OH, -CO₂H, -OB(OH)₂, -OH, -CH₃, -NH₂ or -N(R¹¹)₂ where each R¹¹ is independently a C₁-C₄ alkyl chain;
   and in which, for formula IV:
      R¹² is H or a C₁-C₄ alkyl chain;
      or a pharmaceutically acceptable salt of the compound of formula I, II, III, or IV.

Preferably the TLR4 agonist is chosen from the group consisting of the chemical compounds identified and exemplified in US 2003/0153532 under the names ER803022, ER803058, ER803732, ER803789, ER804053, ER804057, ER804058, ER804059, ER804442, ER804764, ER111232, ER112022, ER112048, ER 112065, ER112066, ER113651, ER118989, ER119327 and ER119328.

The compounds can be in the form of diastereoisomers or in a racemic form (mixture of diastereoisomers) when the chemical structure comprises several asymmetrical carbons. For example, ER804057 and ER804053, which have 4 asymmetrical carbons, are diastereoisomers of ER112066, which is the racemic form. ER804057 is in an (R,R,R,R)-type isomeric configuration, whereas ER804053 is in an (R,S,S,R)-type configuration. Similarly, ER804058, which is in an (R,R,R,R)-type isomeric configuration, and ER804059, which is in an (R,S,S,R)-type isomeric configuration, are diastereoisomers of ER113651, which is the racemic form. ER803022, which is in an (R,R,R,R)-type configuration, ER803732, which is in an (R,S,S,R) configuration, and ER803789, which is in an (R,R,S,R) configuration, are also diastereoisomers of one and the same chemical molecule. The diastereoisomers which have an R,R,R,R-type configuration, which are generally more active than the other forms, are preferably used. Among these, ER804057 is particularly preferred. It is dodecanoic acid *(1R,6R,22R*,*27R)*-1,27-diheptyl-9,19-dihydroxy-9,19-dioxido-14-oxo-6,22-bis[(1,3-dioxotetradecyl)amino]-4,8,10,18,20,24-hexaoxa-13,15-diaza-9,19-diphosphaheptacosane-1,27-diyl ester; it is in the form of a free acid or in the form of a salt. The molecular weight of the free acid form is 1579, that of the disodium salt is 1624. The empirical formula of the disodium salt is C₈₃H₁₅₈N₄Na₂O₁₉P₂.

Another example of TH-1 adjuvant comprises QS21, an HPLC purified non-toxic fraction derived from the bark of Quillaja Saponaria Molina. Optionally this may be admixed with 3 De-O-acylated monophosphoryl lipid A (3D-MPL) or the like. The method of production of QS21 is disclosed in US 5,057,540. A combination of QS21 with cholesterol or a derivative thereof is a useful combination as it decreases the side effects of QS21 (WO 96/33739) and may be also used as TH-1 adjuvant.

Further adjuvants which are preferential stimulators of TH1-type response include immunomodulatory oligonucleotides, for example unmethylated CpG sequences as disclosed in WO 96/02555 or TH-1 cytokines such as IFNγ, IL-2, IL-12, IL-18.

Combinations of different TH1 stimulating adjuvants, such as those mentioned hereinabove, are also contemplated as providing an adjuvant which is a preferential stimulator of TH1-type response. For example, QS21 can be formulated together with 3D-MPL. The ratio of QS21:3D-MPL will typically be in the order of 1:10 to 10:1; preferably 1:5 to 5:1 and often substantially 1:1. The preferred range for optimal synergy is 2.5:1 to 1:1 3D-MPL:QS21.

The supplementary adjuvants, as cited above, or any other supplemental component of the emulsion can be introduced into the O/W emulsion compositions of the present invention, for example, (a) if in oil-soluble or oil-dispersible form, by adding the additional component to the oil phase (s) or (b) if in water-soluble or water-dispersible form, by adding the additional component to the aqueous phase, either before or after emulsification. For supplementary adjuvant added after emulsification, mention may be made of QS21/3D-MPL mixture that is added to an O/W emulsion comprising squalene, alpha tocopherol and Tween 80 as described in WO 95/17210. Typically for human administration, QS21 and 3D-MPL can be present in the vaccine composition in the range of 1 µg-200 µg, such as 10-100 µg, or even in the range of 10 µg-50 µg per vaccine dose. Usually, the O/W emulsion comprises from 2 to 10% squalene, from 2 to 10% alpha tocopherol and from 0.3 to 3% tween 80. The ratio of squalene: alpha tocopherol is often equal to or less than 1 as this provides a more stable emulsion. Span 85 may also be present at a level of 1%.

Where the components of the O/W emulsion allow the production of a thermoreversible O/W emulsion as mentioned above, it is also possible to apply the process described in WO 2007/080308, to produce a thermoreversible O/W emulsion containing a TLR4 agonist like those that have one of the chemical structure mentioned above (I, II, III, or IV).

Alternatively, the adjuvant may be administered concurrently with the vaccine composition containing the gB HCMV in an O/W emulsion of the present invention, either by extemporaneous addition of the adjuvant to the vaccine composition just prior to the administration or in separate compositions. The adjuvant may also be administered prior or subsequent to the vaccine composition of the present invention.

The gB of HCMV in the vaccine composition of the invention is usually in the form of a mixture in an O/W emulsion or in a thermoreversible O/W emulsion, each of the mixtures containing the TH2-1 adjuvant, as the case may be, the mixture being ready to be administered. The vaccine composition can be prepared by the simple addition of an O/W emulsion or a thermoreversible O/W emulsion to a solution of gB in an appropriate buffer or vice versa by the addition of a solution of gB in an appropriate buffer to an O/W emulsion or a thermoreversible O/W emulsion. In case the vaccine composition contains a TH-1 adjuvant, it may be incorporated into the O/W emulsion or into the gB solution before the extemporaneous mixing of the two solutions. The mixture is then gently swirled and then stored until administration.

In another embodiment, the vaccine composition of the invention is prepared extemporaneously, just before administration to the human subjects. Thus, the invention provides kits including the various components ready for mixing. The kit allows the O/W emulsion or the thermoreversible O/W emulsion component, the gB of HCMV component, and optionally the adjuvant component to be kept separately until the time of use.

The components are physically separate from each other within the kit, and this separation can be achieved in various ways. For instance, the O/W emulsion component and the gB component may be in two separate containers, such as vials. The contents of the two vials can then be mixed, e.g., by removing the content of one vial and adding it to the other vial, or by separately removing the contents of both vials and mixing them in a third container. In a preferred arrangement, one of the kit components is in a syringe and the other is in a container such as a vial. The syringe can be used (e.g., with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another preferred arrangement, the two kit components are held together but separately in the same syringe, e.g., a dual-chamber syringe. When the syringe is actuated (e.g., during administration to a patient) the contents of the two chambers are mixed. This arrangement avoids the need for a separate mixing step at the time of use. The kit components will generally be in aqueous form. In some arrangements, a component (the gB of HCMV component or the O/W emulsion as the case may be) is in dry form (e.g., in a lyophilized form), with the other component being in aqueous form. The two components can be mixed in order to reactivate the dry component and give an aqueous composition for administration to a patient. A lyophilized component can be located within a vial or in a syringe. Dried components may include stabilizers such as mannitol, sucrose, or dodecyl maltoside, as well as mixtures thereof e.g. lactose/sucrose mixtures, sucrose/mannitol mixtures, etc.

The disclosure provides a kit comprising: (i) a first kit component comprising a gB of HCMV and (ii) a second kit component comprising an O/W emulsion or a thermoreversible O/W emulsion and the use of such kit for preventing HCMV infection.

The amount of HCMV gB antigen to be administered to the human subjects is from 5µg to 100µg, and more preferably from 10µg to 100µg. The vaccine composition is at least to be administered two times within 1 to 2 month interval between each administration by the SC (subcutaneous), ID (intradermal), or IM (intramuscular) route to the HCMV seronegative human subjects. A third immunization within a 4 to 6 month interval after the second administration is preferable. A preferred scheme of vaccination comprises the administration by the IM route at 0, 1 and 6 months, 3 doses of 20µg gB antigen in an O/W emulsion. A fourth administration may be useful as a booster vaccine dose. Preferably, the gB antigen is under the form of gBdTM and the O/W emulsion is a MF59-like emulsion, such as the MF59^{™} emulsion.

### 1: Design of the clinical study

### Study population

A Phase II, modified double-blind, randomized, placebo-controlled study was conducted to determine whether a recombinant gB subunit vaccine in an O/W emulsion called MF59^{™} can prevent maternal HCMV infection in a high risk population of HCMV seronegative mothers aged 14 to 40 years who were recruited from postpartum wards and from the community. They were screened for susceptibility to HCMV using a standard commercial IgG antibody test (Axsym® HCMV IgG -Abbott Diagnostics).

Only HCMV seronegative young mothers that were at 6 weeks and not more than 12 months postpartum and who met inclusion and exclusion criteria were included in the study. They were to be given by the IM Route in the left deltoid at 0, 1 and 6 months either 3 doses of the HCMV gB vaccine with MF59^{™} emulsion (20 µg gB per 0.50 mL dose) or a placebo (saline). The clinical trial started in 1999 and the enrollment was completed in April 2006. total number of 441 young mothers of 21 years old (mean range) were given at least one immunization: 225 young mothers were given at least one vaccine dose and 216 young mothers were given at least one placebo dose.

### Composition of the vaccine

The gB was a recombinant glycoprotein, which was produced in Chinese hamster ovary (CHO) cell cultures. The gB gene from the Towne strain of HCMV was mutagenized to remove the cleavage site and the transmembrane portion of the molecule in order to facilitate secretion in cell culture. The secreted molecule was a polypeptide of 807 amino acids, retaining 19 potential N-linked glycosylation sites, and is also called gBdTm. The purification process involved affinity and ion-exchange chromatography steps.

The HCMV gB vaccine was provided as two separate components (the gB antigen and the MF59^{™} emulsion) contained in two separate vials which were combined prior to administration:
- one vial containing the HCMV gB (liquid sterile solution)
- one vial containing the MF59^{™} (liquid sterile emulsion)
Both products were stored refrigerated between +2 and +8°C.

To prepare the HCMV gB/MF59^{™} vaccine the purified gB (0.35 ml) was mixed with MF59^{™} (0.35 ml) immediately prior to administration as follows: just prior to vaccine administration, the HCMV gB antigen vial and the MF59^{™} vial were removed from the refrigerator and brought to room temperature by hand-warming. Using a 1.0 mL tuberculin syringe, 0.35 mL of the MF59^{™} emulsion was withdrawn and transferred to the HCMV gB antigen vial. The mixture was then swirled gently for 10 seconds. To administer the vaccine, 0.5 mL were withdrawn from the final mixture with a new needle and injected intramuscularly.

Each dose of the formulated vaccine that was administered contained 20 µg of purified gB and 9.75 mg of MF59^{™} (the dose of MF59^{™} was expressed as the squalene content) and the excipients listed in the following table.

| **Component** | **Quantity per dose (0.5 mL)** |
|---|---|
| HCMV gB | 0.02 mg |
| Sodium Chloride | 3.94 mg |
| L-(+) Histidine | 0.39 mg |
| EDTA, disodium salt, dihydrate | 0.28 mg |
| Dibasic Sodium Phosphate | 0.29 mg |
| Monobasic Sodium Phosphate | 0.06 mg |
| Sodium Citrate, dihydrate | 0.66 mg |
| Citric Acid, monohydrate | 0.04 mg |
| Squalene | 9.75 mg |
| Polysorbate 80 (Tween 80) | 1.18 mg |
| Sorbitan trioleate (Span 85) | 1.18 mg |
| Water for injections | q.s. to 0.50 mL |

### Follow up

Women were followed every 3 months and for 3 years from the third immunization. Any participant that received at least one dose of the study vaccine (or the placebo) and was CMV antibody negative on the day the first dose of vaccine was given, was considered to be in the intent to treat population (ITT). Women that were given the 3 vaccine doses were included in the per protocol population (PP). The primary endpoint was the time to HCMV infection, demonstrated by the presence of HCMV specific antibodies not directed to gB, and/or by the shedding of the virus in blood, urine, or saliva swabs and/or by PCR detection of HCMV. The secondary objectives were the evaluation of the immunogenicity, safety of the vaccine, and the rate of congenital HCMV infection in the offspring of immunized women. With regard to the safety of the vaccine, it was noted that the vaccine induced, as usually observed for vaccines, more local reactions than the placebo (mainly more pain at injection site and more indurations). There was no significant difference in the reporting of systemic reactions between the vaccine and placebo groups, except for myalgia that were more frequently reported in the vaccine group (between 11 and 16% of the women in the vaccine group had myalgia compared to 3 to 7% in the placebo group). On the overall local and systemic reactions observed, the vaccine was considered to be safe and well tolerated.

### Detection of HCMV infection in the study population and offspring

Blood samples were collected at each follow-up study visit (every 3 months). Serum was separated from whole blood by centrifugation; an aliquot was used for seroconversion studies whereas another aliquot was prepared for immunogenicity antibody assays.

HCMV seroconversion was assessed using a HCMV IgG antibody assay (Axsym HCMV IgG, Abbott Diagnostics) with pre-absorption of serum with purified HCMV gB. This gB absorbed HCMV IgG assay described by Zhang C. et al (Vaccine, 23:507-510 (2004)) accurately identified subjects with natural immunity to HCMV from infection, but remained negative in subjects with immunity induced exclusively by HCMV gB vaccine. It had therefore the great advantage of not revealing whether trial participants received vaccine; results were negative unless HCMV infection occurred. A gB absorbed HCMV IgG antibody level of ≥5 AU/mL (antibody units/mL) was considered a positive screen for HCMV infection.

All subjects who had a positive screen for HCMV infection were asked to return for monthly follow-up for 6 months: at these visits blood was collected and the subject was asked to provide urine, mouth swab and vaginal swab for virus culture. HCMV infection was confirmed by either of the following: 1) A positive viral culture or positive polymerase chain reaction (PCR) test for HCMV DNA from any site or 2) Serum antibody to recombinant HCMV proteins, not including gB detected by western blot immunoassay. The western blot immunoassay was only performed on samples from subjects who were positive with the gB absorbed HCMV IgG assay and had no positive HCMV cultures or PCR results. Immunoblot results were considered positive and HCMV infection confirmed when a serum collected after HCMV infection was suspected, showed clear reactivity with 2 or more HCMV proteins other than gB. At the termination visit, all subjects had urine collected for HCMV culture and for PCR detection of HCMV DNA.

Newborns born to mothers participating in this vaccine trial were tested for HCMV infection by a virus culture of urine and/or a mouth swab.

### 2. Results

From the 464 subjects that were enrolled, a total number of 441 subjects in the ITT population were available for efficacy analysis: 225 in the vaccine group and 216 in the placebo group.

The numbers of subjects who acquired a HCMV infection in each group within the 42 months follow up following the first administration of the product are summarized in table I.

**Table I: Number of HCMV infection cases in the ITT population according to the regimen followed (based on data collected until June 2007)**

| | Total | Vaccine | Placebo |
|---|---|---|---|
| Number of subjects evaluable | 441 | 225 | 216 |
| Number of subjects with a confirmed HCMV infection | 49 | 18 | 31 |

Confirmed HCMV infection cases were identified as those for which positive results were observed both on a gB adsorbed HCMV IgG assay and by PCR, virus culture or western blot immunoassay.

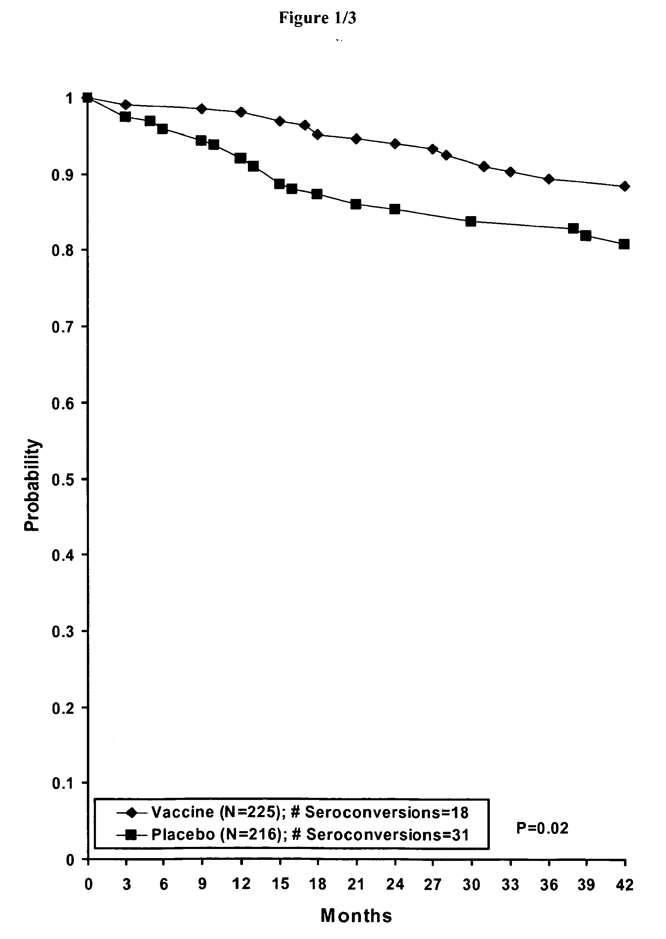

The timing of the HCMV infection events (defined as the date of the first positive result) that occurred in the ITT population is summarized in table II and represented in figure 1.

**Table II: Timing of HCMV infection events in the ITT population within the 42 months of follow up (based on data collected until June 2007)**

| Time post enrollment | Number of HCMV events in the ITT population according to the regimen followed | |
|---|---|---|
| | Vaccine group (225) | Placebo (216) |
| 0-6 months | 2 | 8 |
| 7-12 months | 2 | 7 |
| 13-18 months | 5 | 8 |
| 19-24 months | 2 | 3 |
| 25-30 months | 2 | 2 |
| 31-36 months | 4 | 0 |
| 37-42 months | 1 | 3 |
| Total number of HCMV events | 18 | 31 |

Cytomegalovirus infection occurred in 31/216 (14.4%) placebo recipients and 18/225 (8%) vaccine recipients. Infection rates were 3.3/100 person-years in the CMV vaccine group and 6.6/100 person-years in the placebo group, an overall vaccine efficacy of 50% (95% CI= 6.5; 73.3)

The p value of the log rank test calculated from the Kaplan Meier curves (p= 0.02) from the number of subjects that acquired HCMV infections in the placebo group as well as in the vaccine group shows a significant difference between the vaccine group and the placebo group (see fig 1). The overall results show that the HCMV vaccine was efficacious to prevent HCMV infection in the ITT population.

The number of subjects at risk of infection at each time interval are indicated in the table III below.

**Table III: number of subjects at risk of infection by month of follow-up (data collected until June 07)**

| **Subjects at Risk by Month of Follow-up** | | | | | | | | | | | | | | | |
|---|---|---|---|---|---|---|---|---|---|---|---|---|---|---|---|
| **Month** | 0 | 3 | 6 | 9 | 12 | 15 | 18 | 21 | 24 | 27 | 30 | 33 | 36 | 39 | 42 |
| **CMVgB** | 225 | 213 | 211 | 204 | 195 | 178 | 160 | 154 | 145 | 136 | 127 | 116 | 112 | 98 | 88 |
| **Placebo** | 216 | 193 | 185 | 178 | 169 | 153 | 141 | 128 | 121 | 114 | 108 | 104 | 97 | 87 | 75 |

The relative efficacy of the HCMV vaccine was further assessed in the 0-12 month period post enrollment, period for which all the women enrolled achieved their one year follow up as of June 2007 (including drop-outs considered until their last day of participation in the study). The relative efficacy of the HCMV vaccine was measured in the ITT population as well as in the PP population. The results obtained are summarized in tables IV and V.

**Table IV: Intent to Treat population: Events and Efficacy by Follow-up Interval**

| Interval | Result | Vaccine | Placebo |
|---|---|---|---|
| 0-6 months | person-years of follow-up | 106.4 | 96.3 |
| | events | 2 | 8 |
| | events/100 person-years of follow-up | 1.88 | 8.30 |
| | efficacy | 77.4% CI95% = [-12.9; 97.7] | |
| | | | |
| 0-12 months | person-years of follow-up | 207.0 | 185.1 |
| | events | 4 | 15 |
| | events/100 person-years of follow-up | 1.9 | 8.1 |
| | efficacy | 76.3 % CI95%[25.7; 94.3] | |
| | | | |

**Table V: Per Protocol population: Events and Efficacy by Follow-up Interval**

| Interval | Result | Vaccine | Placebo |
|---|---|---|---|
| 7-12 months | person-years of follow-up | 85.83 | 72.75 |
| | events | 2 | 6 |
| | events/100 person-years of follow-up | 2.33 | 8.28 |
| | efficacy | 71.7% CI95% [-58.0; 97.2] | |
| | | | |

CI 95% calculation is based on the Poisson exact method for ratios, with adjustment on the unbalanced time of follow-up between both groups.

These results clearly show that the vaccine had a very good efficacy in the two populations at least for a one year period after the first dose of the vaccine was given (vaccine efficacy is greater than 70%). The design of this vaccine was shown to be especially suitable for HCMV seronegative mothers who had infants or toddlers since their infants or toddlers by themselves could represent an important risk factor of HCMV transmission to their mothers.

The data regarding the number of HCMV infection cases in the ITT population according to the regimen followed, the timing of HCMV infection events in the ITT population within the 42 months of follow up and the relative efficacy of the HCMV vaccine in the ITT population by follow up interval on a 24 month period were further analyzed one year later from all the data collected until August 2008. The updated results are summarized in tables VI, VII and VIII

**Table VI: Number of HCMV infection cases in the ITT population according to the regimen followed (based on data collected until August 2008)**

| | Total | Vaccine | Placebo |
|---|---|---|---|
| Number of subjects evaluable | 441 | 225 | 216 |
| Number of subjects with a confirmed HCMV infection | 60 | 24 | 36 |

**Table VII: Timing of HCMV infection events in the ITT population within the 42 months of follow up (based on data collected until August 2008)**

| Time post enrollment | Number of HCMV events in the ITT population according to the regimen followed | |
|---|---|---|
| | Vaccine group (225) | Placebo (216) |
| 0-6 months | 2 | 8 |
| 7-12 months | 2 | 7 |
| 13-18 months | 6 | 8 |
| 19-24 months | 3 | 6 |
| 25-30 months | 3 | 2 |
| 31-36 months | 5 | 0 |
| 37-42 months | 3 | 5 |
| Total number of HCMV events | 24 | 36 |

**Table VIII: Intent to Treat population: Events and Efficacy by Follow-up Interval (based on data collected until August 2008)**

| Interval | Result | Vaccine | Placebo |
|---|---|---|---|
| 0-6 months | person-years of follow-up | 106.3 | 95.8 |
| | Events | 2 | 8 |
| | events/100 person-years of follow-up | 1.88 | 8.35 |
| | Efficacy | 77.4% CI95% = [-12.9; 97.7] | |
| | | | |
| | | | |
| 0-12 months | person-years of follow-up | 207.0 | 185.1 |
| | Events | 4 | 15 |
| | events/100 person-years of follow-up | 1.9 | 8.1 |
| | Efficacy | 76.3 % CI95%[25.7 ; 94.3] | |
| | | | |
| 0-24 months | Person-years of follow-up | 385.3 | 344.1 |
| | Events | 13 | 29 |
| | Events/100 person-years of follow-up | 3.37 | 8.43 |
| | Efficacy | 59.9% CI95% [20.5 ; 80.9] | |
| | | | |

The data regarding the relative efficacy of the HCMV vaccine in the PP population by follow up intervals on a 24 month period were further analyzed one year later from all the data collected until August 2008. The updated results are summarized in table IX

**Table IX: Per Protocol population: Events and efficacy by Follow-up Interval**

| Interval | Result | Vaccine | Placebo |
|---|---|---|---|
| 7-12 months | person-years of follow-up | 85.83 | 72.75 |
| | events | 2 | 6 |
| | events/100 person-years of follow-up | 2.33 | 8.28 |
| | efficacy | 71.7% CI95% [-58.0; 97.2] | |
| | | | |
| 7-24 months | person-years of follow-up | 241.8 | 202.1 |
| | events | 10 | 16 |
| | events/100 person-years of follow-up | 4.14 | 7.90 |
| | efficacy | 47.7% CI95% [-22.4 ; 78.8] | |
| | | | |

The updated results are similar to those previously shown and confirm the efficacy of the vaccine in the prevention of HCMV infection in young mothers.

Neutralizing antibody rates were measured in a subgroup of 50 non-infected mothers having received three doses of the vaccine (D0, D30 and D180). The procedure used was the following:
Test serum samples and control serum samples were routinely tested at an initial dilution of 1:4. Test and control samples were added to the appropriate wells of the test plate and 2-fold serial dilutions of each serum sample were run in duplicate. Sixty microliters of virus solution (containing a fixed quantity of CMV towne strain virus and guinea pig complement) was dispensed into each sample well of a test plate except for the serum control wells, cell control wells, culture medium control wells, or cell titration wells. The test plates were incubated at 37°C + 5% CO₂ in a humidified incubator for 60-90 minutes. After incubation, a MRC-5 cell suspension was adjusted at a cell concentration of 3 x 10⁵ cells/ml in culture medium. One hundred microliters of the MRC-5 cell suspension was added to all of the wells except for the culture medium control wells and the cell titration wells. Cell titration wells received 100 µl of undiluted, 1/2, 1/4, 1/8, 1/16, 1/32, 1/64, or 1/128 dilutions of the MRC-5 cell suspension. Culture medium control wells received 220 µl of culture medium alone. The test plates were then incubated at 37°C + 5% CO₂ in a humidified incubator for 44-52 hours. They were finally observed microscopically to determine if each well contained healthy uninfected MRC-5 cells or if cytopathic effect (CPE) was present indicating that the MRC-5 cells were infected with CMV. The titer of neutralizing antibodies was calculated for each serum sample tested and was figured as the inverse of the highest dilution where no CPE was observed in the wells of the test plate. The neutralizing antibody titer was considered as being significant when it was at least of 4. As negative controls, serum samples of uninfected mothers from the placebo group were tested for their neutralizing activity and as expected the neutralizing antibody titers were permanently found below 4.
Figure II shows the evolution of the geometric mean titer of neutralizing antibodies during the 42 months follow-up in a subgroup of 50 non-infected mothers having received three vaccine doses. The GMT titer is higher than 120 fourteen days after the third injection, which is far more than the titer of neutralizing antibodies observed in HCMV naturally infected women (The GMT is around 50 using the same procedure).
Figure III shows the percentage of mothers with neutralizing antibodies (i.e. neutralizing antibody titer is at least 4) during the 42 months follow-up among the 50 non-infected mothers having received three vaccine doses. It is noteworthy that 100% of the mothers have neutralizing antibodies 14 days after the third injection and that the persistence of neutralizing antibodies along the time is effective since more than 60% of the mothers still have neutralizing antibodies 3 years after the third injection

The Cellular Mediated Immunity response (CMI) based on the lymphocyte proliferative assay was also tested during the 42 month follow-up study in the subgroup of non-infected mothers having received three doses of the vaccine and compared with that observed in a subgroup of uninfected mothers from the placebo group

A standard lymphocyte proliferation assay was used to measure cell mediated immune response to CMV gB and other antigens; this assay reflected primarily CD4 T-lymphocyte proliferation in response to incubation with antigen. Peripheral blood mononuclear cells (PBMC) were isolated from whole blood (diluted 1:1 with phosphate buffered saline) by centrifugation for 25 minutes at 2000 RPM at 4° C. on Histopaque 1044. The PBMC were carefully aspirated from the interface and washed twice in Hank's balanced salt solution and once in RPMI media. The cell count is adjusted to 1.5 x 10⁶ per ml in complete media (RPMI 1640 + L-glutamine, 10% fetal calf serum, with penicillin and streptomycin). Aliquots of 0.2 ml (300,000) cells were dispensed into wells of a 96 well round bottom tissue culture plate. Triplicate wells were used for each antigen and for blank (negative control). Antigens, CMB gB, CMV AD169 whole virus antigen, and staphlococcal enterotoxin B (used as a universal positive stimulant) were dispensed into triplicate wells for each PBMC sample. The tissue culture plates were then incubated for 5 days at 37° C in 5% CO₂ in a humidified incubator. On day 5, tritiated thymidine was added to each well and cells were incubated for an additional 24 hours. Cells were harvested onto filter strips which were dispensed into vials containing liquid scintillation cocktail; radioactivity was counted in a liquid scintillation counter. The stimulation index was calculated as the ratio of counts per minute (cpm) with the test antigen divided by cpm of the blank. The results are summarized in table X

**Table X: Lymphocyte proliferative responses observed during the 42 month follow-up in non-infected mothers having received three vaccine doses and in non-infected mothers having received three placebo doses**

| Time post enrollment (in months) | Vaccine | | placebo | |
|---|---|---|---|---|
| | Number of mothers tested | Median of S.I (range) | Number of mothers tested | Median of S.I. (range) |
| 9 | 48 | 8.9 (0.8-79.3) | 41 | 1.2 (0.4-3.4) |
| 18 | 64 | 10.2 (0.9-55.3) | 51 | 1.3 (0.4-3.5) |
| 27 | 63 | 7.89 (1.0-75.1) | 49 | 1.2 (0.4-7.7) |
| 42 | 59 | 6.94 (0.7-60.3) | 38 | 1.1(0.3-9.4) |

| | | | | |
|---|---|---|---|---|
| S.I. means Stimulating index | | | | |

The results show that there is no CMI response observed in mothers who received 3 placebo doses since the median of the S.I is around 1 whatever the time post enrollment tested. On the contrary, a consistent and persistent CMI is observed in study participants who received 3 vaccine doses since the median of the S.I is significantly higher. This surprisingly shows that the vaccine, although it does not contain internal protein of HCMV, is able to induce a persistent cellular immunity which may also contribute to the prevention of HCMV infection on the long term.

As of August 2008, of the 441 Intent To Treat subjects, 201 had one or more pregnancies during the study (91 in the vaccine group and 110 in the placebo group) during the 42 months of follow-up after the first injection of the vaccine seven participants in the placebo group got HCMV infected during pregnancy compared with 1 in the vaccine group.

As of August 2008, there were 93 live newborns born during the study period to mothers in the vaccinated group, all of them being free of congenital infection, whereas 5 live newborns of a total number of 112 live newborns born to mothers in the placebo group were infected at birth as indicated in table XI.

**Table XI: Number of Congenital CMV infections observed in live newborns according to the regimen followed by the mothers (based on the data collected until August 2008)**

| | Vaccine | Placebo |
|---|---|---|
| Live newborns | 93 | 112 |
| Numbers of HCMV Congenital infection at birth* | 0(0%) | 5 (4.5%) |

| | | |
|---|---|---|
| *: Congenital infection is established when the virus is present in the culture of newborns' mouth swabs. | | |

The 2-tailed P value of the Fisher's exact test is 0.06

This result clearly indicates that the HCMV vaccine according to the invention is able to prevent HCMV congenital infections.

## Claims

1. A recombinant gB subunit vaccine composition containing the recombinant gB antigen of Human Cytomegalovirus (HCMV) in an oil in water (O/W) emulsion for use in a method to prevent HCMV infection in a HCMV seronegative human subject.

2. A vaccine according to claim 1 for the use according to claim 1, wherein the seronegative human subject is a HCMV seronegative child-bearing-age woman.

3. A vaccine according to claim 1 for the use according to claim 1, wherein the seronegative human subject is a HCMV seronegative mother.

4. A recombinant gB subunit vaccine composition containing the recombinant gB antigen of Human Cytomegalovirus (HCMV) in an oil in water (O/W) emulsion for use in a method to prevent HCMV congenital infection in a newborn.

5. A vaccine according to claim 4 for the use according to claim 4, wherein the newborn is born to a HCMV seronegative child-bearing-age woman.

6. A vaccine according to claim 4, for the use according to claim 5, wherein the HCMV seronegative child-bearing-age woman is a HCMV seronegative mother.

7. A vaccine according to claim 1 or 4 for the use according to any of claims 1 to 6, wherein the gB antigen is the sole antigen of HCMV in the vaccine composition.

8. A vaccine according to claim 1 or 4 for the use according to any of claims 1 to 7, wherein the gB antigen of HCMV further comprises one or several mutations on the cleavage site.

9. A vaccine according to claim 1 or 4 for the use according to any of claims 1 to 8, wherein the gB antigen of HCMV is a full length gB polypeptide, a full length gB polypeptide lacking substantially all the trans membrane domain, a full length gB polypeptide lacking substantially all the intracellular domain, or a full length gB polypeptide lacking substantially both the transmembrane domain and the intracellular domain.

10. A vaccine according to claim 1 or 4 for the use according to any of claims 1 to 9, wherein the gB antigen of HCMV is gBdTm.

11. A vaccine according to claim 1 or 4 for the use according to any of claims 1 to 10, wherein the O/W emulsion is a MF59-like emulsion.

12. A vaccine according to claim 1 or 4 for the use according to any of claims 1 to 11, wherein the O/W emulsion further comprises a TH-I adjuvant.

13. A vaccine according to claim 1 or 4 for the use according to any of claims 1 to 12, wherein the gB antigen of HCMV is to be administered to the human subject in an amount of 5µg to 100 µg.

14. A vaccine according to claim 1 or 4 for the use according to any of claims 1 to 13, wherein the vaccine composition is a ready to be administered formulation, wherein the gB antigen and optionally the TH-1 adjuvant is already mixed within the O/W emulsion.

15. A vaccine according to claim 1 or 4 for the use according to any of claims 1 to 13, wherein the vaccine composition is prepared extemporaneously by mixing the O/W emulsion with the gB antigen and optionally with the TH-1 adjuvant prior to administering to the HCMV seronegative human subject.

16. A vaccine according to claim 1 or 4 for the use according to any of claims 1 to 15, wherein the vaccine composition is to be administered at least two times within 1-2 month interval between each administration by the subcutaneous (SC), intradermal (ID) or intramuscular (IM) route.

17. A vaccine according to claim 1 or 4 for the use according to claim 16, wherein the vaccine composition is to be administered a third time within a 4 to 6 month interval between the second administration and the third administration.

18. A vaccine according to claim 1 or 4 for the use according to claim 17, wherein the vaccine composition is to be administered a fourth time as a booster dose.

## Patentansprüche

1. Rekombinante gB-Spaltimpfstoffzusammensetzung, die das rekombinante gB-Antigen des Human-Cytomegalievirus (HCMV) in einer Öl-in-Wasser-(O/W)-Emulsion enthält, für die Verwendung in einem Verfahren zur Verhinderung von HCMV-Infektion in einem HCMV-seronegativen menschlichen Patienten.

2. Impfstoff nach Anspruch 1 für die Verwendung nach Anspruch 1, wobei es sich bei dem seronegativen menschlichen Patienten um eine HCMV-seronegative Frau im gebärfähigen Alter handelt.

3. Impfstoff nach Anspruch 1 für die Verwendung nach Anspruch 1, wobei es sich bei dem seronegativen menschlichen Patienten um eine HCMV-seronegative Mutter handelt.

4. Rekombinante gB-Spaltimpfstoffzusammensetzung, die das rekombinante gB-Antigen des Human-Cytomegalievirus (HCMV) in einer Öl-in-Wasser-(O/W)-Emulsion enthält, für die Verwendung in einem Verfahren zum Vorbeugen gegen angeborene HCMV-Infektion in einem Neugeborenen.

5. Impfstoff nach Anspruch 4 für die Verwendung nach Anspruch 4, wobei das Neugeborene das Kind einer HCMV-seronegativen Frau im gebärfähigen Alter ist.

6. Impfstoff nach Anspruch 4 für die Verwendung nach Anspruch 5, wobei es sich bei der HCMV-seronegativen Frau im gebärfähigen Alter um eine HCMV-seronegative Mutter handelt.

7. Impfstoff nach Anspruch 1 oder 4 für die Verwendung nach einem der Ansprüche 1 bis 6, wobei das gB-Antigen das einzige HCMV-Antigen in der Impfstoffzusammensetzung ist.

8. Impfstoff nach Anspruch 1 oder 4 für die Verwendung nach einem der Ansprüche 1 bis 7, wobei das HCMV-gB-Antigen weiterhin eine oder mehrere Mutationen an der Spaltstelle umfasst.

9. Impfstoff nach Anspruch 1 oder 4 für die Verwendung nach einem der Ansprüche 1 bis 8, wobei es sich bei dem HCMV-gB-Antigen um ein Volllängen-gB-Polypeptid, um ein Volllängen-gB-Polypeptid, dem im Wesentlichen die gesamte Transmembrandomäne fehlt, um ein Volllängen-gB-Polypeptid, dem im Wesentlichen die gesamte intrazelluläre Domäne fehlt, oder um ein Volllängen-gB-Polypeptid, dem im Wesentlichen sowohl die Transmembrandomäne als auch die intrazelluläre Domäne fehlen, handelt.

10. Impfstoff nach Anspruch 1 oder 4 für die Verwendung nach einem der Ansprüche 1 bis 9, wobei es sich bei dem HCMV-gB-Antigen um gBdTm handelt.

11. Impfstoff nach Anspruch 1 oder 4 für die Verwendung nach einem der Ansprüche 1 bis 10, wobei es sich bei der O/W-Emulsion um eine MF59-artige Emulsion handelt.

12. Impfstoff nach Anspruch 1 oder 4 für die Verwendung nach einem der Ansprüche 1 bis 11, wobei die O/W-Emulsion weiterhin ein TH-1-Adjuvans umfasst.

13. Impfstoff nach Anspruch 1 oder 4 für die Verwendung nach einem der Ansprüche 1 bis 12, wobei das HCMV-gB-Antigen dem menschlichen Patienten in einer Menge von 5 µg bis 100 µg zu verabreichen ist.

14. Impfstoff nach Anspruch 1 oder 4 für die Verwendung nach einem der Ansprüche 1 bis 13, wobei es sich bei der Impfstoffzusammensetzung um eine verabreichungsfertige Formulierung handelt, wobei das gB-Antigen und gegebenenfalls das TH-1-Adjuvans bereits mit der O/W-Emulsion vermischt ist.

15. Impfstoff nach Anspruch 1 oder 4 für die Verwendung nach einem der Ansprüche 1 bis 13, wobei die Impfstoffzusammensetzung durch Vermischen der O/W-Emulsion mit dem gB-Antigen und gegebenenfalls mit dem TH-1-Adjuvans ad hoc vor der Verabreichung an den HCMV-seronegativen menschlichen Patienten hergestellt wird.

16. Impfstoff nach Anspruch 1 oder 4 für die Verwendung nach einem der Ansprüche 1 bis 15, wobei die Impfstoffzusammensetzung mindestens zwei Mal mit einem Zeitabstand von 1-2 Monaten zwischen jeder Verabreichung auf dem subkutanen (s.c.), intradermalen (i.d.) oder intramuskulären (i.m.) Weg zu verabreichen ist.

17. Impfstoff nach Anspruch 1 oder 4 für die Verwendung nach Anspruch 16, wobei die Impfstoffzusammensetzung ein drittes Mal mit einem Zeitabstand von 4 bis 6 Monaten zwischen der zweiten Verabreichung und der dritten Verabreichung zu verabreichen ist.

18. Impfstoff nach Anspruch 1 bis 4 für die Verwendung nach Anspruch 17, wobei die Impfstoffzusammensetzung ein viertes Mal in Form einer Auffrischungsdosis zu verabreichen ist.

## Revendications

1. Composition vaccinale sous unitaire à protéine gB recombinante contenant l'antigène gB recombinant du cytomégalovirus humain (HCMV) dans une émulsion d'huile dans l'eau (O/W) pour utilisation dans une méthode pour prévenir une infection à HCMV chez un sujet humain séronégatif pour HCMV.

2. Vaccin selon la revendication 1 pour l'utilisation selon la revendication 1, **caractérisé en ce que** le sujet humain séronégatif est une femme en âge de procréer séronégative pour HCMV.

3. Vaccin selon la revendication 1 pour l'utilisation selon la revendication 1, **caractérisé en ce que** le sujet humain séronégatif est une mère séronégative pour HCMV.

4. Composition vaccinale sous unitaire à protéine gB recombinante contenant l'antigène gB recombinant du cytomégalovirus humain (HCMV) dans une émulsion d'huile dans l'eau (O/W) pour utilisation dans une méthode pour prévenir une infection congénitale à HCMV chez un nouveau-né.

5. Vaccin selon la revendication 4 pour l'utilisation selon la revendication 4, **caractérisé en ce que** le nouveau-né est né d'une femme en âge de procréer séronégative pour HCMV.

6. Vaccin selon la revendication 4, pour l'utilisation selon la revendication 5, **caractérisé en ce que** la femme en âge de procréer séronégative pour HCMV est une mère séronégative pour HCMV.

7. Vaccin selon la revendication 1 ou 4 pour l'utilisation selon l'une quelconque des revendications 1 à 6, **caractérisé en ce que** l'antigène gB est le seul antigène de HCMV dans la composition vaccinale.

8. Vaccin selon la revendication 1 ou 4 pour l'utilisation selon l'une quelconque des revendications 1 à 7, **caractérisé en ce que** l'antigène gB de HCMV comprend en outre une ou plusieurs mutations sur le site de clivage.

9. Vaccin selon la revendication 1 ou 4 pour l'utilisation selon l'une quelconque des revendications 1 à 8, **caractérisé en ce que** l'antigène gB de HCMV est un polypeptide gB entier, un polypeptide gB entier dépourvu substantiellement de tout le domaine transmembranaire, un polypeptide gB entier dépourvu substantiellement de tout le domaine intracellulaire, ou un polypeptide gB entier dépourvu substantiellement à la fois du domaine transmembranaire et du domaine intracellulaire.

10. Vaccin selon la revendication 1 ou 4 pour l'utilisation selon l'une quelconque des revendications 1 à 9, **caractérisé en ce que** l'antigène gB de HCMV est gBdTm.

11. Vaccin selon la revendication 1 ou 4 pour l'utilisation selon l'une quelconque des revendications 1 à 10, **caractérisé en ce que** l'émulsion O/W est une émulsion de type MF59.

12. Vaccin selon la revendication 1 ou 4 pour l'utilisation selon l'une quelconque des revendications 1 à 11, **caractérisé en ce que** l'émulsion O/W comprend en outre un adjuvant TH-1.

13. Vaccin selon la revendication 1 ou 4 pour l'utilisation selon l'une quelconque des revendications 1 à 12, **caractérisé en ce que** l'antigène gB de HCMV est destiné à être administré au sujet humain dans une quantité de 5 µg à 100 µg.

14. Vaccin selon la revendication 1 ou 4 pour l'utilisation selon l'une quelconque des revendications 1 à 13, **caractérisé en ce que** la composition vaccinale est une formulation prête à être administrée, **caractérisé en ce que** l'antigène gB et éventuellement l'adjuvant TH-1 sont déjà mélangés dans l'émulsion O/W.

15. Vaccin selon la revendication 1 ou 4 pour l'utilisation selon l'une quelconque des revendications 1 à 13, **caractérisé en ce que** la composition vaccinale est préparée de façon extemporanée en mélangeant l'émulsion O/W avec l'antigène gB et facultativement avec l'adjuvant TH-1 avant administration au sujet humain séronégatif pour HCMV.

16. Vaccin selon la revendication 1 ou 4 pour l'utilisation selon l'une quelconque des revendications 1 à 15, **caractérisé en ce que** la composition vaccinale est destinée à être administrée au moins deux fois dans un délai de 1 à 2 mois entre chaque administration par voie sous-cutanée (SC), intradermique (ID) ou intramusculaire.

17. Vaccin selon la revendication 1 ou 4 pour l'utilisation selon la revendication 16, **caractérisé en ce que** la composition vaccinale est destinée à être administrée une troisième fois dans un intervalle de 4 à 6 mois entre la deuxième administration et la troisième administration.

18. Vaccin selon la revendication 1 ou 4 pour l'utilisation selon la revendication 17, **caractérisé en ce que** la composition vaccinale est destinée à être administrée une quatrième fois en tant que dose de rappel.
